# EUROPEAN PATENT SPECIFICATION

(11) **EP 4 490 491 B1**
(45) Date of publication and mention of the grant of the patent: **19.08.2026**
(21) Application number: 23710694.3
(22) Date of filing: 08.03.2023
(51) Int. Cl.: G01N 15/1429, C07K 17/06, G01N 15/1433, G01N 33/92, G06N 3/0464, G06N 3/09, G06N 20/00, G06T 7/62, G06T 7/64, G01N 33/50, G01N 33/58, G01N 21/64, G06T 7/00, G06V 20/69

(54) **METHOD FOR CHARACTERISING EXTRACELLULAR VESICLES BY SMLM MICROSCOPY**
VERFAHREN ZUR CHARAKTERISIERUNG EXTRAZELLULÄRER VESIKEL DURCH SMLM-MIKROSKOPIE
PROCÉDÉ DE CARACTERISATION DE VESICULES EXTRACELLULAIRES PAR SMLM-MICROSCOPIE

(30) Priority: 09.03.2022 GB 202203276
(43) Date of publication of application: 15.01.2025
(73) Proprietor: Oxford Nanoimaging Limited, Oxford OX2 8TA (GB)
(72) Inventor: JING, Bo, Oxford Oxfordshire OX2 8TA (GB); FELCE, James Hannes, Oxford Oxfordshire OX2 8TA (GB); MIKLOSI, Andras Gabor, Oxford Oxfordshire OX2 8TA (GB)
(74) Representative: Mewburn Ellis LLP
(86) International application number: PCT/EP2023/055843
(87) International publication number: WO 2023/170118

(56) References cited:
- US-A1- 2005 207 633
- HUILIN SHAO ET AL: "New Technologies for Analysis of Extracellular Vesicles", CHEMICAL REVIEWS, vol. 118, no. 4, 31 January 2018 (2018-01-31), US, pages 1917 - 1950, XP055700486, ISSN: 0009-2665, Retrieved from the Internet <URL:https://pubs.acs.org/doi/10.1021/acs.chemrev.7b00534> [retrieved on 20230601], DOI: 10.1021/acs.chemrev.7b00534
- ZHANG JUNLI ET AL: "Localized fluorescent imaging of multiple proteins on individual extracellular vesicles using rolling circle amplification for cancer diagnosis", JOURNAL OF EXTRACELLULAR VESICLES, vol. 10, no. 1, 11 November 2020 (2020-11-11), UK, pages 1 - 16, XP093051154, ISSN: 2001-3078, Retrieved from the Internet <URL:https://pubmed.ncbi.nlm.nih.gov/33304477/> [retrieved on 20230601], DOI: 10.1002/jev2.12025

## Description

### FIELD OF THE INVENTION

The present application relates to methods, system and apparatus for characterising Extracellular Vesicles (EVs) through fluorescence and/or high resolution imaging.

### BACKGROUND

Extracellular vesicles are of increasing importance in the areas of diagnostics, therapeutics, and biological discovery.

To identify the nature, source, and/or potential diagnostic/therapeutic benefit of an EV population, there are four key characteristics which can be measured.

A first key characteristic is the size of the EVs. Currently EVs are categorised based on their diameter since this is the most reliable method for distinguishing EVs produced by the three different classical mechanisms: exosomes (30-150 nm), microvesicles/ectosomes (50-1000 nm), and apoptotic bodies (100-2000 nm).

A second key characteristic is surface biomarker composition. Molecules (proteins, glycans, nucleic acids, lipids, and other metabolites) present on the surface of EVs determine their targeting to appropriate tissues for cargo delivery, and also exert effects directly through interaction with cell-surface receptors (e.g. PDL1-bearing) or scavenging of soluble factors (e.g. chemokine receptor-bearing EVs).

A third key characteristic is the presence and type of luminal content of the EV - in other words, the characteristics of the material enclosed/held within the EV. Molecules transported within the EV lumen are frequently released into target cells and so can have substantial effects on cellular responses. This is a highly active area of research for the delivery of therapeutics.

A fourth key characteristic is the integrity of the EVs - in other words, whether the EVs remain intact. Intact EVs contain at least both lipid and protein components, which distinguishes them from particulate protein aggregates (including deliberate assemblies such as supramolecular attack particles) and protein-free liposomes.

Currently, the most sensitive methods for characterising these characteristics are based on the bulk analysis of large numbers of EVs. This ranges from global, 'omics' approaches (such as proteomics, transcriptomics, genomics, lipidomics and metabolomics) to more targeted analyses of specific molecules using immune detection or RNA-specific PCR-like approaches. Analytical technologies and assays for these two levels of EV analysis differ, and require different levels of pre-processing and purification. Although highly sensitive and relatively straightforward to perform, these experiments do not provide any information at the single-EV level, and all details are lost about e.g. population heterogeneity, distinct subpopulations, and correlations between different biomarkers. Moreover, characterisation of the bulk protein content of EVs is frequently challenging due to the high potential for sample contamination by irrelevant soluble or aggregated proteins. This also makes normalisation of protein content relative to known EV proteins extremely difficult to interpret.

A more informative alternative is to perform characterisation at the single-EV level, such that population heterogeneity is not masked. Currently, methods to achieve this focus on a limited subset of physical parameters (e.g. concentration, size, morphology) and/or their biochemical content (e.g. proteins, nucleic acids, lipids and metabolites), however there is no single technology that suits most or all analytical parameters. Moreover, researcher confidence in currently available technologies is often low. This is reflected in the official guidelines from a number of international organisations in the EV field, which recommend the orthogonal use of several complementary analyses to confirm results (see EV-TRACK Consortium et al. and Théry *et al.* - details for which are given in the references section at the end of this description).

One key consideration is that pre-isolation and purification of EVs is essential for many analyses in order to avoid interference of non-EV contaminants, however this is likely to lead to significant loss of EVs in addition to potential morphological damage. Moreover, isolation procedures (which are typically based on ultracentrifugation) are generally biased towards EVs of specific size and/or density.

In an attempt to circumvent this issue, some characterisation methods include in-assay capture steps for EVs of interest through targeting of specific surface-presented biomarkers. Typically such capture-based assays use the (so-called) general EV surface tetraspanin markers (CD9, CD63 and CD81) for capture; e.g. with time-resolved immunoassays (Duijvesz, D et al) surface plasmon resonance (Rikkert, L.G. *et al*.), or interferomic/diffraction limited imaging (Daaboul, G.G. *et al.*) including commercial systems such as ExoView^{®}.

However, it is increasingly evident that only limited fractions of EVs carry these 'general' EV markers in abundance. Their expression is highly influenced by EV source and physiological state of parent cells. It is not clear what represents the critical density of these markers sufficient to allow capture, nor how this might be affected by other factors, e.g. the density of the EV glycocalyx, or tetraspanin clustering.

On top of this, current technologies for the analysis of individual EVs are typically low-throughput, and highly dependent on fine experimental detail, making them inconsistent and prone to user variance.

One medium-throughput approach is nano-flow cytometry, which is able to rapidly analyse large numbers of EVs within a sample, but suffers from poor sensitivity for labelled biomarkers and indirect size determination based on weak light scattering. Thus, although it is technically a single-EV approach, interpretation of results is generally only reasonably confident at the population level, and even then with the caveat that low density biomarkers will be difficult to detect. This is a substantial limitation as the physiological relevance of biomarkers with low numbers on EVs can be very high, such that the presence of even one molecule can be biologically meaningful. Similarly, having high confidence that an EV is completely negative for a given biomarker can also be extremely important, which is not possible when sensitivity is too low to discriminate zero copies from a small number of copies. Probing the cargo using nano-flow cytometry is also hindered by various technical difficulties which include aggregation, disruption and loss of cargo due to the usage of detergents.

Other approaches use microscopy. The most prominently used microscopy method for EVs currently is electron microscopy, which is typically seen as the gold standard for determination of EV size. This, however, is extremely low-throughput, is dependent on highly specialised expertise and equipment, and provides only limited biomarker-specific information.

Conventional diffraction-limited fluorescence microscopy has been utilised in a number of approaches, such as the ExoView^{®} system available from NanoView, wherein EVs are captured based on tetraspanin expression, probed for biomarkers of interest using fluorescently conjugated antibodies, and then imaged as diffraction-limited spots. This provides some information about the biomarker composition of the EV, however it cannot report EV morphology or biomarker spatial distribution due to the inherent resolution of the approach. It also does not allow accurate confirmation that the particles analysed are intact EVs, as the lack of spatial information means contaminant particles such as protein aggregates cannot be reliably distinguished from full EVs. This is reflected in the broadly low level of confidence across the EV field for any single analytical tool. Most crucially, it is of low sensitivity for low-abundance biomarkers due to the poor detection efficiency of conventional fluorescence microscopy for individual molecules, and so typically reports the presence or absence of biomarkers over a given threshold rather than absolute quantification. As discussed above for nano-flow cytometry, the poor ability to discriminate between low copy numbers and zero copies is also highly problematic, and means many biological insights are inaccessible using this approach. The classification of EVs into positive and negative populations for a given biomarker is not meaningful if the threshold for detection is above the threshold for biological relevance of the biomarker. Given the very low threshold for biological relevance in EVs (even 1-2 molecules), any technique that aims to achieve comprehensive characterisation in a meaningful sense must have clear discrimination between truly negative and simply low copy number EVs.

In view of the above, there remains a need to develop improved methods, system and apparatus for characterising vesicles which are able to provide a more complete and accurate picture of EVs at a single-EV level, at reasonable throughput speeds. Related to this, there is also a need for improved methods for capturing vesicles to facilitate their analysis and characterisation.

Shao H, et al (Chem. Rev. 2018, 118, pages 1917-1950) discloses an overview of analytical technologies for EV detection and their clinical applications, including using fluorescence imaging techniques.

Zhang et al (J. Extracell. Vesicles, 2020, 10, page 1-16) discloses localized fluorescent imaging of multiple proteins on individual extracellular vesicles using rolling circle amplifcation for cancer diagnosis.

US 2005/207633 discloses methods of classifying cells into subpopulations using cell classifying data.

### SUMMARY OF THE INVENTION

In view of the above, the present disclosure provides a method of characterising vesicles comprising:
(1) a sample preparation step, comprising providing a test specimen with vesicles attached to a substrate, wherein the vesicles are labelled with one or more fluorescent probes;
(2) an image acquisition step, comprising imaging the vesicles on the test specimen using single molecule localisation microscopy (SMLM) of said one or more fluorescent probes to generate image data including SMLM image data;
(3) an image processing step, comprising: calculating position data for individual fluorescent probes on the test specimen based on the SMLM image data;
   using the position data to identify individual vesicles; and
   calculating at least three characterising parameters for individual vesicles from the position data and/or image data, at least one of the characterising parameters being a morphological parameter, and
   constructing a feature vector for individual vesicles from the characterising parameters;
(4) a data transformation step, comprising inputting the feature vectors for individual vesicles into a dimensionality reduction algorithm to calculate modified feature vectors of lower dimensionality for individual vesicles; and
(5) a characterisation step, involving characterising each vesicle by comparing the modified feature vector for that vesicle against other modified feature vectors obtained for other vesicles from the test specimen or from reference data.

Advantageously, this approach allows different populations of vesicles *within* a sample to be distinguished and characterised, instead of producing a single "sample-wide" assessment. Furthermore, the analysis is based on multiple parameters for each individual vesicle including a morphological parameter, but reduces this to a more manageable modified feature vector.

The at least one morphological parameter may be assessed, for example, through single molecule localisation microscopy (SMLM) of the one or more fluorescent probes and/or through a scattering-based technique, such as interferometric scattering microscopy (iSCAT). Preferably, the at least one morphological parameter is assessed by SMLM.

The characterising parameters may include, for example, copy number of the fluorescent probes associated with an individual vesicle. This may be obtained, for example, through a super resolution fluorescence microscopy technique, such as SMLM, or through a diffraction-limited microscopy technique such as photobleaching step analysis (PBSA).

For example, in one embodiment steps (2) and (3) may comprise carrying out a scattering-based measurement to determine the size of the vesicles (said morphological parameter), and fluorescence-based imaging to determine the copy number through photobleaching step analysis (PBSA).

In especially preferred embodiments, steps (2) and (3) comprise carrying out SMLM to determine both said morphological parameter and the copy number of the fluorescent probes associated with an individual vesicle.

Suitably, the vesicles are extracellular vesicles.

The invention is defined by a method of characterising vesicles comprising:
(1) a sample preparation step, comprising providing a test specimen with vesicles attached to a substrate, wherein the vesicles are labelled with one or more fluorescent probes;
(2) an image acquisition step, comprising imaging the vesicles on the test specimen using single molecule localisation microscopy (SMLM) of said one or more fluorescent probes to generate image data including SMLM image data;
(3) an image processing step, comprising:
   calculating position data for individual fluorescent probes on the test specimen based on the SMLM image data;
   using the position data to identify individual vesicles; and
   calculating at least three characterising parameters for individual vesicles from the position data and/or image data, at least one of the characterising parameters being a morphological parameter (the morphological parameter preferably being derived from said position data for individual fluorescent probes associated with a given vesicle), and
   constructing a feature vector for individual vesicles from the characterising parameters;
(4) a data transformation step, comprising inputting the feature vectors for individual vesicles into a dimensionality reduction algorithm to calculate modified feature vectors of lower dimensionality for individual vesicles; and
(5) a characterisation step, involving characterising each vesicle by comparing the modified feature vector for that vesicle against other modified feature vectors obtained for other vesicles from the test specimen or from reference data.

The SMLM technique may be, for example, (direct) stochastic optical reconstruction microscopy [(d)STORM], photoactivated localisation microscopy (PALM), or point accumulation for imaging in nanoscale topography (PAINT) microscopy.

Advantageously, the use of SMLM to identify the precise location of fluorescent probes can provide a wealth of information about the vesicles which is poorly characterised by, or sometimes inaccessible to, conventional microscopy techniques.

Firstly, in instances where the fluorescent probes are used to label biomarkers present on a vesicle, SMLM is able to identify with high confidence whether the vesicle is negative for a given biomarker or simply contains that biomarker at a low concentration. In contrast, in conventional methods used to characterise EVs it can be difficult or impossible to discriminate between zero copies and a small number of copies of a particular biomarker. Secondly, accumulated SMLM data for a given vesicle can be used to identify the boundary of the vesicle, and hence the size and morphology, even when the vesicle is smaller than the diffraction limit of the illumination light. In contrast, for conventional fluorescence microscopy it is not possible to obtain reliable or detailed size or morphology information.

Thirdly, the high precision accuracy of SMLM data can be used to identify the distribution of fluorescent probes across the surface, allowing information to be gained about clustering and/or colocalisation.

Advantageously, all of this information can be gained using a single test specimen and (generally) the same imaging system - facilitating sample preparation and reducing the time and effort to obtain results.

Turning now to the way in which the data is processed, transformation of the characterising parameters into a modified feature vector helps subsequent analysis in the characterisation step.

Taken together, the method of the present invention can allow comprehensive measurement of the structural, physical and compositional features of individual vesicles, whilst synthesising these measurements into a small number of dimensions for ease of interpretation and understanding.

This is in contrast, for example, to the SMLM analysis described in Saliba *et al,* where measurement of the size of vesicles is carried out on a separate set of vesicles compared to those used for measurement of biomarker distribution measurements, and no attempt is made to carry out higher dimensional analysis across multiple parameters for individual vesicles.

The technique also stands in contrast to the method taught in Han *et al.* where different fractions of EVs obtained by centrifugation or SEC were labelled with fluorescently-tagged tetraspanin molecules and examined by conventional (diffraction limited) fluorescence microscopy. The individual EVs were characterised according to whether they were positive or negative (in other words, a binary measure) for each of the different markers, and this information combined to assess the co-presence of the markers. The authors then carried out multidimensional analysis to characterise the co-presence of the markers, but this was done at a sample level (in respect of different fractions) instead of a single vesicle level. In addition, similarly to Saliba et al., size information was obtained by SEM/SPM on a separate set of vesicles to those used for the fluorescence measurements. Such a technique necessarily loses potentially important information about variation between vesicles in a sample, as well as high-resolution information about the distribution of molecules on/in the EV. It also necessitates separate sample preparation techniques to prepare the sample for size measurement compared to those for fluorescence measurements.

Finally, the technique should also be contrasted with that taught in Lee *et al.* In that instance, EVs were again labelled with fluorescently-tagged tetraspanin molecules and examined by conventional (diffraction limited) fluorescence microscopy. Fluorescence intensity data were recorded across multiple colour channels for individual vesicles. The fluorescence intensity data was then subjected to multidimensional analysis using t-distributed stochastic neighbour embedding (t-SNE). Separately, again, size information was obtained by SEM on a separate set of vesicles to those used for the fluorescence measurements. However, as with the method in Han et al., the method taught in Lee et al. does not permit simultaneous measurement of EV size and indeed requires an entirely separate sample separation to determine EV size, and also provides no information about the absolute or relative distribution of molecules on/in the EV.

Suitably, steps (3)-(5) are implemented using a computer with suitable software installed thereon. In certain embodiments, these and other steps may be implemented on hardware, software or a combination of hardware and software (i.e., firmware). Moreover, these and other steps may be implemented locally (at the imaging device), at a remote server or both.

Suitably, a computer implemented system is configured to carry out steps (2)-(5).

For example, the invention may be implemented by a system or apparatus configured to:
- obtain image data of one or more fluorescent probes on vesicles immobilised on a substrate;
- identify individual vesicles in the image data, and calculate at least three characterising parameters for individual vesicles from the image data, at least one of the characterising parameters being a morphological parameter,
- construct a feature vector for individual vesicles from the characterising parameters;
- input the feature vectors for individual vesicles into a dimensionality reduction algorithm to calculate modified feature vectors of lower dimensionality for individual vesicles; and
- characterise each vesicle by comparing the modified feature vector for that vesicle against other modified feature vectors obtained for other vesicles from the test specimen or from reference data.

Suitably, an exemplary method of the invention comprises (6) a diagnostic step, in which the output from the characterisation step is used to form a clinical picture. For example, the output from the characterisation step may be used to identify the presence/absence of a disease state, and/or the nature of a disease state. Alternatively, the output from the characterisation step may be used to identify the physiological origin of the vesicles.

### Step (1) - Sample preparation

The characterisation method of the present invention is carried out on vesicles attached to a substrate.

### Substrate

The substrate may be any substrate compatible with fluorescence imaging. In this regard, the substrate should be at least partially transparent to light of at least one of the following wavelengths: 405 nm, 473 nm, 488 nm, 532 nm, 561 nm, 638 nm, 640 nm, 710 nm and/or 850 nm.

The substrate should preferably have a planar surface or a surface of which at least a portion is planar and to which planar surface or planar portion the vesicles are bound.

The substrate may be a microscope slide, optical fibre, or prism. The term "microscope slide" also extends to, for example, coverslips, and situations where a microscope slide is incorporated as part of a larger structure, such as a microtiter plate (e.g. a 6, 24, 96, 384 or 1536-well microtiter plate or at least a portion thereof, preferably the bottom of a well thereof) or a microfluidic chamber (e.g. in a microfluidic chip).

Preferably the substrate comprises, consists essentially of or consists of glass or an optically transparent polymer. Most preferably the substrate comprises, consists essentially of or consists of glass.

### Immobilisation

Suitably, the sample preparation step comprises immobilising the vesicles on the substrate. This ensures that image data acquired over time does not need to account for movement of the vesicles across the substrate during the course of imaging.

Immobilisation of the vesicles may be achieved through any suitable technique.

Optionally, immobilising the vesicles comprises contacting the substrate with a vesicle-containing sample and adsorbing the vesicles onto the substrate. The vesicles may adsorb directly to the substrate. To enhance such direct adsorption, the substrate may be activated, e.g. through hydroxylation of the surface (e.g. with piranha solution, other strong oxidising agents, or plasma etching), or addition of charged/polar functional group. Alternatively, the substrate may include a coating to enhance adsorption of the vesicles. Such a coating may be, for example, a positively charged polymer, such as poly-L-lysine, chitosan, or nitrocellulose. These positively charged polymers are able to attract the predominantly negatively charged headgroups of EV membranes.

Optionally, immobilising the vesicles comprises contacting the substrate with a vesicle-containing sample and reacting the vesicles with the substrate surface itself. For example, the substrate may incorporate functional groups which react with moieties on the surface of the vesicles. Suitable functional groups may include, for example, aldehyde functionality introduced to the substrate.

Preferably, immobilising the vesicles comprises providing the surface of the substrate with one or more binding agents, and contacting the substrate with a vesicle-containing sample such that the vesicles bind to the binding agents.

Immobilising the vesicles through the use of binding agents, instead of relying on adsorption, has a number of advantages.

Firstly, adsorption-based strategies can result in high levels of unwanted impurities from the vesicle-containing sample adsorbing to the substrate alongside vesicles, particularly in instances where charged coatings (such as poly-L-lysine) are applied to the surface. These impurities can produce high levels of background fluorescence which complicate analysis (particularly for SMLM), as well as potentially weakening the adherence of the vesicles to the substrate surface. In contrast, the use of binding agents can limit the amount of these impurities binding to the surface.

Secondly, adsorption-based immobilisation can result in reagents used in the methods of the invention (e.g. the one of more fluorescent probes) being deposited on the substrate in greater quantities than with binding-agent-based immobilisation. It is important to minimise this type of unwanted non-specific binding of reagents to achieve maximum signal to noise, particularly for SMLM measurements.

Preferably, the binding agent is selected to specifically bind biomarkers on the vesicle surface. In such instances, suitable binding agents include, for example, an antibody, aptamer, nucleic acid, polypeptide, or a purified or synthetic ligand. The antibody may be, for example, a monoclonal antibody, a polyclonal antibody, or an antibody fragment such as a F(ab')₂, F(ab)₂, Fab', Fab, variable fragment (Fv), single chain variable fragment (scFv), diabodies, linear antibodies, single-chain antibody molecules, and multispecific antibodies formed from antibody fragments.

The binding agent may target any suitable molecule provided on the vesicle surface. For example, the binding agent may bind to a lipid, a protein, a glycosylation motif attached to a glycolipid, glycoprotein or a proteoglycan, or a metabolite present on the vesicle surface.

Optionally, the binding agent may be chosen to select vesicles of a certain type from a broader pool of vesicle types in a sample. In other words, the immobilising step may be used as a means of identifying/purifying the sample.

In such instances, the binding agent targets a biomarker found on only a sub-set of the vesicles. The biomarker may be associated with a particular disease state. For example, the binding agent may target one or more glypicans, such as glypican-1, glypican-2, glypican-3, glypican-4 glypican-5, and glypican-6, most preferably glypican-1 or glypican-3, as taught in WO 2017/136676. Glypicans may be indicative of cancer.

Alternatively, the binding agent may be chosen to bind a broad spectrum of vesicle types. In this regard, the methods of the invention incorporating SMLM are particularly well suited to examining samples containing a broad spectrum of vesicle types, because the use of SMLM allows a large range of characterising parameters to be extracted for each vesicle which can be used to distinguish between vesicle types with high confidence and accuracy.

The strategies described below are well-suited to capturing a broad spectrum of vesicle types, through targeting species common to vesicles in general.

Preferably, the binding agent is a TIM protein such as TIM-1, TIM-3 or TIM-4, most preferably TIM-4 (sometimes referred to as TIMD-4). Methods for obtaining TIM proteins and using them to capture vesicles is taught in, for example US 2018/0120299 .

TIM-4 binds to phosphatidylserine (PS) in vesicles membranes, and the present inventors have found it to be an especially useful way to attach a broad spectrum of vesicles to the surface.

Alternatively, the binding agent may be an antibody (including any of the antibody variant structures mentioned above) or aptamer which binds to a tetraspanin, MHC class I, MHC class II, HSP70, Annexin V, Flotillin or EpCAM. Preferably, the antibody or aptamer binds to a tetraspanin. Suitable tetraspanins include, for example, CD9, CD63, CD81, and CD82 which are transmembrane biomarkers known to be present in the membranes of vesicles. Amongst these, CD9, CD63 and CD81 are especially preferred. Thus, the binding agent may be, for example, an anti-CD9 antibody, an anti-CD63 antibody, an anti-CD81 antibody, or an anti-CD82 antibody.

Alternatively, the binding agent may be a lectin. The lectin may be, for example, a mannose-binding lectin, a fucose-binding lectin, a galactose-binding lectin, an N-acetylglucosamine-binding lectin, N-acetylgalactosamine-binding lectin, a sialic acid binding lectin, or a glucose-specific lectin. Suitable lectins include, for example, Particularly preferred examples of lectins for use in the invention include Wheat Germ Agglutinin (WGA), Galanthus nivalis Lectin (GNA), Aleuria aurantia lectin (AAL), or Maackia amurensis lectin (MAA).

Alternatively, the binding agent may be or include a curvature-sensing peptide. Examples of suitable curvature-sensing peptides for vesicle immobilisation are taught in, for example, Gori, A. et al and WO 2020/1044381. For example, the curvature-sensing peptide may be a peptide or peptidomimetic derived from bradykinin, myristoylated alanine rich c-kinase substrate (MARCKS), synaptotamin, arfgap1, magainin, α-synuclein, synapsin (I), endophilin, c-reactive protein or amphyphysin, as taught in WO 2020/1044381. Preferably, the curvature sensing-peptide is derived from bradykinin, for example the motif RPPGFSPFR, as taught in Gori, A. *et al.*

More generally, any binding agent which may be immobilised on the capture surface that binds specifically to a biomolecule found on the vesicles' surface or is exposed to the vesicles' surface may be used as a means of capture (post translational modification on proteins, nucleic acids, lipids other than the ones mentioned above or amino acid sidechains). In addition the surface charge of EVs may be used as a means of capture.

In another approach, the binding agent may bind to a capture moiety introduced to the vesicle surface. The capture moiety may be introduced by reacting the vesicle surface with a suitable compound containing the capture moiety. Alternatively, the capture moiety may be introduced to the vesicle surface through incubating the vesicles with a lipid containing said capture moiety, to allow the lipid to incorporate into the vesicle membrane.

For example, biotin may be introduced to the vesicle surface to allow capture of the vesicles using a biotin-binding molecule such as avidin/streptavidin/neutravidin. Optionally, the vesicles may be directly biotinylated, for example through reacting the vesicles with sulfo-NHS-biotin, as taught in the immobilisation method of Han *et al.* Alternatively, and more preferably, biotinylated-lipids are introduced into the vesicles. For example, the vesicles may be incubated with biotinylated palmitate, which inserts into the vesicle membrane.

The skilled reader will understand that references to avidin and streptavidin above and below also extend to modified versions of avidin/streptavidin, including deglycosylated variants of avidin such as neutravidin, extravidin, or neutralite, and mono-, di-, tri- or more generally multi-valent versions.

The binding agent may be attached to the substrate through any suitable interaction. For example, the binding agent may be adsorbed to the surface, or may be directly or indirectly bonded to the surface.

Optionally, the binding agent is attached to the substrate through interaction between complementary moieties on the binding agent and the substrate or another compound provided on the substrate. Suitable interactions for this purpose include interactions between biotin and avidin/streptavidin/neutravidin and their variants (including mono-, di-, tri-, or tetra-valent versions), streptag-strep-tactin interactions, Fluorescein Isothiocyanate (FITC)-Anti FITC ab interactions, Digoxigenin (DIG)- Anti DIG ab interactions, Nickel-NTA interactions, Copper-NTA interactions, Maleimide group - sulfhydryl group interactions, N-hydroxysuccinimide (NHS) ester-amine interactions, thiol-thiol interactions or alkyne- azide interactions (including, but not limited to, copper-catalysed alkyne-azide cycloaddition (CuAAC), ruthenium-catalysed alkyne-azide cycloaddition (RuAAC), strain-promoted alkyne-azide cycloaddition (SPAAC)), aldehyde-amine interactions (optionally followed by amine reduction), aldehyde-hydrazine interactions, amine-tetrazine interactions, Staudinger ligation.

In a particularly preferred implementation, the substrate is passivated. In other words, the substrate is subjected to treatment with a passivation agent. Passivation of the surface helps to reduce nonspecific binding of impurities and reagents to the surface of the substrate.

The passivation agent may be, for example, a protein (e.g. bovine serum albumin (BSA) or human serum albumin (HSA)), a nonionic surfactant (such as polysorbate 20 (e.g. Tween^{®}-20), Triton X-100, or poloxamer 407 (e.g. Pluronic^{™} F127)), polyethylene glycol (PEG) (optionally in the form of an ester), or any mixture thereof.

Optionally, the binding agent is attached to a passivation agent. For example, the binding agent may be attached to the substrate via said passivation agent. Attachment of the binding agent to the passivation agent may be achieved through interaction between complimentary moieties provided on the binding agent and passivation agent - e.g. through an anchor moiety on the passivation agent which interacts with a capture moiety on the binding agent. Suitable interactions for this purpose include, for example, interactions between biotin and avidin/streptavidin/neutravidin and their variants (including mono-, di-, tri-, or tetra-valent versions), streptag-strep-tactin interactions, Fluorescein Isothiocyanate (FITC)-Anti FITC ab interactions, Digoxigenin (DIG)- Anti DIG ab interactions, Nickel-NTA interactions, Copper-NTA interactions, Maleimide group - sulfhydryl group interactions, N-hydroxysuccinimide (NHS) ester-amine interactions, thiol-thiol interactions or alkyne- azide interactions (including, but not limited to, copper-catalysed alkyne-azide cycloaddition (CuAAC), ruthenium-catalysed alkyne-azide cycloaddition (RuAAC), strain-promoted alkyne-azide cycloaddition (SPAAC)), aldehyde-amine interactions (optionally followed by amine reduction), aldehyde-hydrazine interactions, amine-tetrazine interactions, Staudinger ligation. The skilled reader will appreciate that in these interactions, one of the compounds may serve as the anchor moiety with the corresponding binding partner acting as the capture moiety - for example, with biotin acting as the anchor moiety and avidin/streptavidin/neutravidin serving as the capture moiety. In a particularly preferred implementation, the interaction is biotin-streptavidin, biotin-neutravidin or biotin-avidin. For example, the binding agent may be biotinylated and the passivation agent may be attached to streptavidin/neutravidin/avidin.

In certain instances, both the passivation agent and binding agent may comprise anchor moieties which interact with a mediating compound, the mediating compound having at least two capture moieties capable of binding to said anchor moieties. For example, both the binding agent and passivation agent may be biotinylated, with their interaction mediated by the addition of streptavidin, neutravidin or avidin (or a variant thereof) as a mediating compound.

In instances where the passivation agent is attached to (a) the substrate and (b) a binding agent through interaction between complimentary moieties, the form of interaction is preferably different for (a) compared to (b). In other words, orthogonal complimentary moieties are used for (a) and (b). This can avoid formation of unwanted species.

Attachment between the binding agent and passivation agent may be carried out prior to exposure to the substrate. However, preferably, the substrate is treated with a passivation agent, with the binding agent subsequently attached to the surface-bound passivation agent. Advantageously, the latter strategy limits the possibility of unwanted aggregates of binding agent and passivation agent forming in instances where one or both of the complimentary moieties are multivalent (e.g. tetrameric avidin or streptavidin).

In certain implementations, the vesicles may first be captured and immobilised on beads using one of the immobilisation strategies above, with those beads subsequently being captured onto the substrate. However, generally the vesicles are immobilised directly on the substrate.

### Exemplary immobilisation strategies

In preferred implementations, immobilising the vesicles comprises:
- treating the substrate with a passivation agent;
- attaching a binding agent (preferably TIM-4) to the passivation agent; and
- attaching the vesicles to the substrate through the binding agent.

In more preferred implementations, immobilising the vesicles comprises:
i. treating the substrate with a passivation agent to bond the passivation agent to the substrate, wherein at least a fraction of the passivation agent comprises an anchor moiety;
ii. treating the substrate with a mediating compound, the mediating compound having multiple capture moieties suitable for binding to said anchor moiety; and
iii. treating the substrate with a TIM protein (preferably TIM-4), the TIM protein having an anchor moiety which binds to said mediating compound.

In such instances, the anchor moiety is preferably biotin, and the mediating compound may be avidin, neutravidin, streptavidin, or a suitable variant thereof. In other words, in an especially preferred implementation, immobilising the vesicles comprises:
i. treating the substrate with a passivation agent to bond the passivation agent to the substrate, and wherein at least a fraction of the passivation agent is biotinylated passivation agent;
ii. treating the substrate with multivalent avidin/neutravidin/streptavidin such that the avidin/neutravidin/streptavidin binds to the biotin moiety of said biotinylated passivation agent; and
iii. treating the substrate with a biotinylated TIM protein (preferably TIM-4) such that the TIM protein binds to the multivalent avidin/neutravidin/streptavidin.

Advantageously, this particular methodology helps to minimise non-specific binding to the surface, and robustly anchors the vesicles to the surface through an interaction which is both specific to a biomarker target (in the case of TIM-4, phosphatidylserine) and applicable to a broad range of vesicles. In addition, the method allows use of only a single type of passivation agent (a fraction of which is biotinylated), and straightforwardly allows only a single passivating step to be carried out.

The ratio of passivation agent comprising said anchor moiety to passivation agent lacking said anchor moiety can be used to control the density of vesicles attached to the surface, as well as the strength of the attachment of vesicles. The ratio of passivation agent comprising said anchor moiety to passivation agent lacking said anchor moiety may be, for example, no more than 0.5:1, no more than 0.4:1, no more than 0.3:1, no more than 0.2:1, no more than 0.1:1, or no more than 0.05:1. In other words, the amount of passivation agent comprising an anchor moiety, as a percentage of the overall amount of passivation agent, may be, for example, 50% or less, 40% or less, 30% or less, 20% or less, 10% or less, or 5% or less.

In the above preferred immobilisation method, the method preferably involves an initial step of treating the substrate with a functionalising chemical to add functional groups to the surface of the substrate, wherein the passivation agent comprises a reactive group which reacts with the said functional groups to bond the passivation agent to the substrate. Preferably, the functionalising chemical is an aminosilane and the reactive group is N-hydroxysuccinimide (NHS) or an ester thereof. The aminosilane may be, for example, 3-aminopropyltriethoxysilane.

Preferably, the passivation agent is PEG, and the biotinylated passivation agent is biotinylated PEG. In especially preferred embodiments, the passivation agent is NHS-PEG, and the biotinylated passivation agent is biotinylated NHS-PEG. The ratio of biotinylated-PEG to non-biotinylated PEG may be no more than 0.5:1, no more than 0.4:1, no more than 0.3:1, no more than 0.2:1, no more than 0.1:1, or no more than 0.05:1. In other words, the amount of biotinylated-PEG as a percentage of the overall amount of PEG passivation agent may be, for example, 50% or less, 40% or less, 30% or less, 20% or less, 10% or less, or 5% or less.

Optionally, the preferred methods above also include cleaning the surface before treating with the functionalizing chemical. Cleaning the surface may comprise treating the surface with piranha solution, plasma, or high pH concentrated salt solution (KOH) treatment.

### Fixation

Preferably, the sample preparation step involves fixing the vesicles prior to the image acquisition step. Fixation may be implemented to eliminate Brownian motion of molecules on the surface of the vesicle, in the cell membrane, or in the lumen of the vesicles. Fixing the sample further helps to immobilise the vesicles on the substrate. This is particularly useful when the vesicles are probed using SMLM, since fixation ensures that individual localisations obtained by SMLM build up a more accurate picture of the boundaries of the vesicles and relative positions of the measured molecules. Regarding the latter, fixation ensures preservation of the supramolecular organisation within the vesicles, such as clustering or segregation of target biomarkers.

Fixation can be performed with any suitable fixative agents. Suitable fixative agents include, for example, formaldehyde, glutaraldehyde, or glyoxal.

A preferred fixative agent is paraformaldehyde, for example, 2% paraformaldehyde in phosphate buffered saline (PBS).

Optionally, any fixation step is followed by a quenching step, in which the vesicles are treated with a quenching agent. This helps to quench any autofluorescent background generated by the fixative agent, and also serves to inactivate unreacted fixative which might otherwise interfere/react with the fluorescent probes. Suitable quenching agents include, for example, glycine (e.g. 1 mM glycine), sodium borohydride and ammonium chloride.

Fixation may be carried out before or after immobilisation of the vesicles on the substrate.

Fixation may be carried out before or after labelling of the vesicles with the one or more fluorescent probes. Preferably, there is a fixation step after labelling, since this can help to retain the fluorescent probes on the vesicles and so allows for sample storage and repeated analysis.

### Permeabilisation

The sample preparation step may involve permeabilisation of the vesicles. For example, the sample preparation step may involve permeabilisation where the one or more fluorescent probes must access the lumen of the vesicles.

Such permeabilization may be carried out after initial fixing of the sample (because fixation helps to maintain vesicle integrity during permeabilization) and before labelling with said one or more fluorescent probes (so that probes can efficiently access the lumen of the vesicles during labelling).

Permeabilization may be carried out by any suitable method, including chemical methods and non-chemical methods.

Chemical methods include the use of detergents, hypertonic solutions to osmotically permeablise the vesicle membrane, the use of pore-forming proteins, or the use of viral spike proteins. Suitable detergents include, for example, Triton-X 100, saponin, digitonin, Tween-20- Tween-80, CHAPS or NP40. The detergents may be used at a quantity of less than 1% (weight/volume), less than 0.5%, less than 0.2%, or less than 0.1%, for example, in a range of 0.001-0.1 % (w/v). Suitable pore-forming proteins include, for example, perforin or granulysin.

Non-chemical methods include, for example, sonication, electroporation, heating or mechanoporation (mechanical disruption).

### Blocking

Optionally, the method involves treating the vesicles with a blocking agent. By "blocking agent" we mean an agent which interacts with the vesicles so as to prevent non-specific binding to the vesicles. Preferably, the blocking agent is applied prior to labelling, such that the blocking agent binds to the vesicles so as to minimise non-specific binding of fluorescent probes to the vesicles, whilst still allowing the one or more fluorescent probes to access the vesicle surface. The blocking agent may be provided as part of a solution, which can be referred to as a "blocking solution".

The blocking agent may be protein based, for example, bovine serum albumin (BSA), human serum albumin (HAS), casein, or collagen, or mixtures of these. The blocking agent may be used, for example, as an aqueous blocking solution with up to 10% w/v of one or more of BSA, HAS, casein or collagen. Preferably, any blocking agent derived from a biological sample such as serum is subjected to purification (e.g. filtration) to remove any vesicles which might otherwise contaminate the sample of interest.

Alternatively, or additionally, the blocking agent may comprise non-fluorescent fluorophore analogues. The non-fluorescent fluorophore analogues may correspond to non-fluorescent versions of the fluorophore present as part of said one or more fluorescent probes. Advantageously, the non-fluorescent fluorophore analogues may bind to sites which would otherwise non-specifically bind to the fluorophores of the one or more fluorescent probes during the labelling step. By "non-fluorescent fluorophore analogues" we mean compounds which are structurally related to fluorophores, but have a modification making them non-fluorescent.

Suitable non-fluorescent fluorophore analogues include, for example, fluorophores which have been subjected to bleaching. Bleaching may be achieved by photobleaching, for example, through exposure of fluorophores to a high intensity excitation source, such as a laser operating at the fluorophores excitation wavelength or a high intensity white light source. Photobleaching may be boosted through use of oxidising agents, such as hydrogen peroxide. In instances where oxidising agents are added to boost photobleaching, excess oxidising agent is preferably neutralised before the blocking agent is applied to the sample. For example, in instances where hydrogen peroxide is used as the oxidising agent excess peroxide may be neutralised through use of catalase.

Other non-fluorescent fluorophore analogues include fluorophores which have been subjected to a chemical modification to prevent fluorescence.

### Labelling

Preferably, the sample preparation step involves labelling the vesicles with said one or more fluorescent probes.

The fluorescent probes may be:
- a fluorescent probe targeting the vesicle membrane; and/or
- a fluorescent probe targeting the vesicle lumen.

The fluorescent probes may be provided in one or more labelling solutions. The labelling solution may be an aqueous solution. All of the fluorescent probes may be incubated with the vesicles as part of a single labelling solution. Alternatively, the fluorescent probes may be incubated separately with different labelling solutions, each containing a different subset (or individual) fluorescent probe type.

At least one of the fluorescent probe types must be compatible with SMLM imaging. However, the method may involve the use of fluorescent probes which are imaged through other methodologies such as non-single molecule imaging.

Optionally, the fluorescent probe is or includes a fluorescently-labelled capture molecule targeting a biomarker, such as an antibody (or any of the variants set out above, including antibody fragments), aptamer, nucleic acid, polypeptide, or a purified or synthetic ligand.

Optionally, labelling is carried out in the presence of a blocking agent to minimise non-specific binding. For example, the sample preparation step may involve (i) a pre-labelling blocking treatment with a blocking solution containing a blocking agent, followed by (ii) a labelling step with a labelling solution containing said blocking agent. In such instances, the concentration of blocking agent in the blocking solution is generally higher than that present in the labelling solution. For example, the blocking solution may contain 5-10% w/v blocking agent, whereas the labelling solution may contain less than 5% w/v.

### Membrane labels

The fluorescent probe targeting the vesicle membrane may be a fluorescent membrane stain, or a fluorescently-labelled capture molecule (such as an antibody or variant thereof, aptamer, nucleic acid, polypeptide, or a purified or synthetic ligand) targeting a biomarker on/in the vesicle membrane.

The biomarker on/in the vesicle membrane may be an integral membrane protein (e.g. a transmembrane or integral monotopic protein) or a peripheral membrane protein. The binding to the biomarker may occur on the exterior surface of the vesicle and/or on the interior surface of the vesicle. Targeting a biomarker on the interior surface of the vesicle typically requires permeabilisation of the vesicle membrane. Suitable permeabilisation techniques are set out above. In instances where a fluorescent probe is used to target the interior membrane surface of the vesicles, the method preferably involves (in order) a first fixation step to fix the vesicle membrane, a permeabilization step, a labelling step in which the fluorescent probe transits the permeabilised membrane, and a second fixation step to fix the fluorescent probe in preparation for imaging.

The fluorescent probe targeting the vesicle membrane may be a carbohydrate-binding probe or a lipid-binding probe.

Suitable carbohydrate-binding probes may be, for example, a mannose-binding probe, a fucose-binding probe, a galactose-binding probe, an N-acetylglucosamine-binding probe, N-acetylgalactosamine-binding probe, a sialic acid binding probe, or a glucose-specific probe. By "mannose-binding probe" we mean a probe with specificity for mannose, or motif incorporating mannose, with analogous definitions applying in respect of the other probe types set out in the preceding sentence. Preferred classes include mannose-binding probes, fucose-binding probes, sialic acid-binding probes, with mannose-binding lectins being particularly preferred.

The carbohydrate-binding probe may be a fluorescently labelled lectin. As the skilled reader will be aware, lectins are proteins that bind to specific carbohydrate structures, but lack enzymatic activity. Advantageously, lectins can have broad binding capability to different vesicles, and are generally readily available from natural sources.

The lectin may be, for example, a mannose-binding lectin, a fucose-binding lectin, a galactose-binding lectin, an N-acetylglucosamine-binding lectin, N-acetylgalactosamine-binding lectin, a sialic acid binding lectin, or a glucose-specific lectin. By "mannose-binding lectin" we mean a lectin with specificity for mannose, or motif incorporating mannose, with analogous definitions applying in respect of the other lectin types set out in the preceding sentence. Examples of suitable lectins are provided in Table 1 below.

**Table 1 - Lectins**

| **Name** | **Specificity** |
|---|---|
| Agaricus bisporus Lectin (ABA/ABL) | Galactoseβ1-3N-Acetylgalactosamine |
| Aleuria aurantia Lectin (AAL) | Fucose |
| Allium sativum Lectin (ASA) | Mannose |
| Amaranthus caudatus Lectin (ACL/ACA) | Galβ3GalNAc |
| Artocarpus integrifolia Lectin (AIA) | Galβ3GalNAc |
| Bauhinia purpurea Lectin (BPL/BPA) | Galβ3GalNAc |
| Concanavalin (ConA) | α- mannose, α-glucose |
| Crotalaria juncea Lectin (CJL) | Gal > GalNAc |
| Datura stramonium Lectin (DSA/DSL) | (N-Acetylglucosamine)2-4 |
| Galanthus nivalis Lectin (GNL/GNA) | α-Mannose |
| Hippeastrum hybrid Lectin (HHL) | α-Mannose |
| Iris hybrid Lectin (IRA) | N-Acetylgalactosamine |
| Lycopersicon esculentum Lectin (LEL/LEA) | (N-Acetylglucosamine)2-4 |
| Maackia amurensis Lectin (MAA/MAL I) | Siaα2-3Galβ1-4GlcNAc |
| Maackia amurensis Lectin (MAA/MAL I+II) | α2-3 Linked Sialic Acids |
| Maackia amurensis Lectin (MAA/MAL II) | Siaα2-3Galβ1-3GalNAc |
| Maclura pomifera Lectin (MPUMPA) | Galβ3GalNAc |
| Morus rubra Lectin (MRL) | Galactose, N-Acetylgalactosamine |
| Narcissus pseudonarcissus Lectin (NPL/NPA) | α-Mannose |
| Psophocarpus tetragonolobus Lectin (PTL/PTA I) | N-Acetylgalactosamine, Galactose |
| Sambucus nigra Lectin (SNA/EBL I) | α2-6 Sialic Acid |
| Sambucus nigra Lectin (SNA/EBL I+II) | Sialic Acid |
| Sambucus nigra Lectin (SNA/EBL II) | N-Acetylgalactosamine > Galactose |
| Solanum tuberosum Lectin (STA/STL) | (N-Acetylglucosamine)2-4 |
| Tulip sp. Lectin (TL) | Complex N-Glycans, Mannose |
| Vicia ervilia Lectin (VEA) | Mannose, Glucose |
| Vicia faba Lectin (VFA) | Mannose |
| Vigna radiate lectin (VRA) | Galactose |
| Wheat Germ Agglutinin (WGA) | N-Acetylgalactosamine, sialic acid |
| Wisteria floribunda Lectin (WFA/WFL) | N-Acetylgalactosamine |

The above lectins are available from companies such as Glycomatrix (Dublin, Ohio, USA).

Preferred classes of lectin include mannose-binding lectins, fucose-binding lectins, sialic acid binding lectins, with mannose-binding lectins being particularly preferred.

Particularly preferred examples of lectins for use in the invention include WGA, Concanavalin A, GNA, AAL, MAA since these lectins bind to motifs found across a broad spectrum of vesicles.

Alternative carbohydrate-binding probes include, for example, cholera toxin subunit B (CTXB, which binds to sialic residues on GM1 gangliosides) bearing a fluorophore.

Examples of lipid-binding probes include, for example, annexin V bearing a fluorophore.

The fluorescent membrane stain may be, for example, a fluorophore bearing a reactive group for reacting with the vesicles' membrane. The reactive group may be, for example, an NHS-ester moiety. In this way, the fluorophore can be covalently bonded to the vesicle surface, and the density readily adjusted through varying the amount of reactive fluorophore introduced during the labelling step.

Alternatively, the fluorescent membrane stain may be a cyanine dye incorporating lipophilic moieties, for example, a long-chain dialkyl carbocyanine dye or dialkylamino styryl dye. Suitable long-chain dialkylcarbocyanines include DiI, DiIC₁₂, DiIC₁₆, DiO, DiOC₁₆, DiD, and DiR, and suitable dialkylaminostyryl dyes include DiA and 4-Di-10-ASP, all available from Thermo Fisher Scientific Corporation. Other suitable membrane stains include, for example, PKH67, sold by Sigma Aldrich, mCLING, Nile Red, BODIPY-cholesterol, or CellMask^{™} stains. Fluorescent membrane stains compatible with SMLM include, for example, CellMask^{™} Orange Plasma Membrane Stain, CellMask^{™} Deep Red Plasma Membrane Stain, and mCLING.

The fluorescent probe targeting the vesicle membrane may be a fluorescently-labelled capture molecule (such as an antibody, aptamer, nucleic acid, polypeptide, or a purified or synthetic ligand) targeting a disease-specific biomarker on/in the vesicle membrane, such as one of those set out below.

### Lumen labelling

Additionally or alternatively the fluorescent probes may include a probe for targeting the lumen of the vesicles. Suitable fluorescent probes include fluorophores capable of being taken up into the lumen and/or fluorescent probes targeting a biomarker present in the lumen - that is, a biomarker which is part of the vesicle's cargo, referred to as a "cargo biomarker". The latter include a capture molecule such as an antibody (or variant thereof), aptamer, nucleic acid, polypeptide, or a purified or synthetic ligand, attached to a fluorophore.

Suitable fluorophores capable of being taken up into the lumen include, for example, carboxyfluorescein succinimidyl ester.

Fluorescent probes for targeting a cargo biomarker may include nucleic acid stains. The nucleic acid stain may be, for example a cyanine dye, for example, the SYTO dyes sold by Thermo Fisher Scientific Corporation and, including blue fluorescent stains (such as SYTO 40, SYTO 41, SYTO 42, SYTO 45), green fluorescent stain such as (SYTO 9, SYTO 10, SYTO 11, SYTO 12, SYTO BC, SYTO 13, SYTO 14, SYTO 16, SYTO 21, SYTO 24, SYTO 25, or SYBR #green I), orange fluorescent SYTO dye (such as SYTO 80-85), and red fluorescent SYTO dye (such as SYTEO 17 or any of SYTO 59-SYTO 64). Other alternatives include SYBR stains (for example SYBR Green I/II), Vybrant DyeCycle stain (such as Vybrant DyeCycle Violet Stain and Vybrant DyeCycle Green Stain), bis-benzimide dyes (a Hoechst stain such as Hoechst 33258, Hoechst 33342 or Hoechst 3458), SYBR Safe, Acridine Orange, and 4'6-diamidino-2-phenylindole (DAPI). Fluorescent nucleic acid stains compatible with SMLM include, for example, SYTOX Green and Syto13.

Biomarkers within the lumen of the vesicles can be fluorescently stained either prior to incorporation into the vesicles (e.g. artificially loaded molecules directly conjugated to fluorophores) or by probing after incorporation.

Introducing fluorescent probes into the lumen of the vesicles typically requires permeabilisation of the vesicle membrane. Suitable permeabilization techniques are set out above. In instances where a fluorescent probe is used to target the lumen of the vesicles, the method preferably involves (in order) a first fixation step to fix the vesicle membrane, a permeabilization step, a labelling step in which the fluorescent probe transits the permeabilised membrane, and a second fixation step to fix the fluorescent probe in preparation for imaging.

### Disease-specific biomarkers

The fluorescent probe(s) may include a fluorescent probe targeting a biomarker associated with a particular disease. For example, the fluorescent probe may target EGFR and/or PD-L1, since elevated levels of these proteins can be indicative of a cancerous state. Other examples of suitable biomarkers are given in Table 2 below.

**Table 2**

| Examples of cancer biomarkers found on/in EVs | |
|---|---|
| **Cancer** | **Markers** |
| Ovarian | L1CAM, CD24, EMMPRIN |
| | TGFβ1, MAGE3/6 |
| | Claudin-4 |
| Glioblastoma | EGFRvIII, EGFR, PDPN IDH1, PD-L1 |
| Melanoma | CD63, Caveolin-1, PDL1 |
| Oral | FasL |
| Gastric | Her-2/neu, CCR6, PD-L1 |
| Bladder | EDIL-3 |
| | LASS2, GALNT1 |
| Kidney | MMP-9, ceruloplasmin, PODXL, DKK4, CAIX, |
| Prostate | ITGA3, ITGB1 |
| | CDCP1, CD151, CD147, PD-L1 |
| Lung | EGFR, PD-L1 |
| Pancreas | Apbb1ip, Aspn, CO31781, Daf2, Foxp1, Gng2 |
| | CD44v6, Tspan8, EpCam, MET, CD104 |
| Leukemia | CD34 |

### Biomarkers of vesicle origin

The fluorescent probe(s) may include a fluorescent probe targeting a biomarker associated with vesicles from a specific origin. The "origin" corresponds, for example, to the type of cell from which the vesicles originated, the status of the cell from which the vesicles originated, or the tissue from which the vesicles originated.

For example, the method has applicability in characterising immune-cell derived vesicles, such as vesicles derived from T-cells or B-cells.

### i. Markers of T-cell origin

In the case of T-cell derived vesicles, the fluorescent probe(s) may target a marker characteristic of T-cell type.

For example, the fluorescent probes may target CD4 and/or CD8, e.g. the fluorescent probes may include a fluorescently labelled capture molecule (e.g. an antibody) specific to CD4 and/or CD8. In instances where the fluorescent probes include a first fluorescent probe targeting CD4 and a second fluorescent probe targeting CD8, the method may be used to characterise the fraction of CD4+ compared to CD8+ vesicles, which is related to the fraction of CD4+ (helper) and CD8+ (cytotoxic) T-cells in the body.

Additionally, or alternatively, the fluorescent probes may be used to target markers of other T-cell populations, such as memory versus effector, naïve versus activated, Th1 versus Th2. For example, the fluorescent probes may target markers of exhaustion (such as PD1, Tim3 and/or LAG3) or markers of effector function (such as CD40L).

Additionally, or alternatively, the fluorescent probes may target the T-cell receptor (TCR), or a specific sub-population of TCRs. With regards to the latter, the fluorescent probes may be TCR allotype-specific capture molecules, such as antibodies or recombinant soluble peptide-MHC complex.

The methods of the invention also have applicability to analysis of vesicles in the context of adoptive cell therapy, such as chimeric antigen receptor T cell (CAR-T) therapy. In such instances, the fluorescent probes may target engineered receptors, such as chimeric antigen receptors (CAR). In such instances, characterisation of the engineered receptors in the vesicles can provide insights into the adoptive cell therapy, such as the expression level of the engineered receptors or colocalization with other markers.

### ii. Markers of B-cell origin

In the case of B-cell derived vesicles, the fluorescent probe(s) may target a marker characteristic of B-cell type.

For example, the fluorescent probe(s) may target a marker indicative of naïve versus memory B-cells, such as CD19 (present in both naïve and memory B-cells) and CD27 (present in memory B-cells but not naïve B-cells).

Additionally, or alternatively, the fluorescent probes may target the B-cell receptor (BCR), or a specific sub-population of BCRs.

### iii. Other cell type origin

Discrimination between other vesicles originating from other cell types, such as neurons or endothelial cells, can also be achieved. The fluorescent probe(s) may target a marker characteristic of that cell type. Examples might include, for example, markers related to Alzheimer's disease, Parkinson's disease or schizophrenia.

### Fluorophores

Fluorophores suitable for use in the fluorescent probes above include dye molecules or fluorescent proteins, for example.

Preferably, in instances where the fluorescent probe is used for SMLM, the fluorescent probe has only a single fluorophore. This minimises double-counting of fluorescent probes during SMLM.

Fluorophores compatible with dSTORM imaging include dyes such as Alexa Fluor^{®} dyes available from Thermo Fisher Scientific Corporation, Atto dyes available from Atto-Tec, cyanine dyes such as Cy3, Cy3B, Cy5 or Cy7 available from Thermo Fisher Scientific Corporation, CF dyes available from Biotium, such as CF488A, CF568 and CF647, DyLight^{®} fluorophores also available from available from Thermo Fisher Scientific Corporation. Preferred examples include Alexa Fluor^{®} 488, Alexa Fluor^{®} 568, Alexa Fluor^{®} 647, ATTO 488, Cy3B, Cy5 or DyLight 550. Particularly preferred examples are (i) ATTO 488, Alexa Fluor^{®} 568, and Alexa Fluor^{®} 647, (ii) CF488, CF568 and CF647; or (iii) Atto488, Cy3b and Alexa Fluor^{®} 647, which can be used in combination due to the spectral separation of their emission.

Fluorophores compatible with PALM imaging include, for example, photoactivatable proteins or photoactivatable dyes. Examples of photoactivatable proteins include, for example, PA-GFP, PA-TagRFP, PA-mCherry1, PA-mKate2; photoconvertible proteins such as PS-CFP2. Kaede, mEos2, mEos3, mEos4b, mMaple3, Dendra2, PSmOrange; and photoswitchable fluorophores such as Dronpa, mGeosM, Dreiklang, mlrisGFP, NijiGFP, with photoactivatable proteins and photoconvertible proteins being preferred. Examples of photoactivatable dyes include, for example, azetidine-substituted dyes, such as the photoactivatable dyes available as part of the Janelia Fluor^{®} range (in particular, PA Janelia Fluor 549 and Janelia Fluor 646), and the Aberrior CAGE range (500, 530, 552, 590 and 635). Preferably, the fluorophores used for PALM are photoactivatable dyes, since these can be used to label endogenous molecules on/in the vesicles.

Fluorophores compatible with PAINT imaging include, for example, dye molecules as taught above, or the use of quantum dots according to the methodology taught in Chang *et al.* The PAINT methodology may be DNA-PAINT or peptide-PAINT, in which case the fluorescent probe is a fluorophore-labelled oligonucleotides, preferably a dye-labelled oligonucleotide. The PAINT methodology may be exchange-PAINT.

As an alternative to labelling with external fluorescent probes, in some embodiments the one or more fluorescent probes may consist of or comprise fluorescent proteins expressed in or on the vesicles, such as (GFP, YFP, RFP and the PALM-compatible variants discussed above), obviating the need for a separate labelling step. However, this approach is generally inapplicable to analysis of samples obtained directly from patients.

In certain implementations, the capture molecule includes a protein tag which binds a fluorophore. For example, the capture molecule may incorporate a HaloTag^{®} or SNAP-Tag^{®} which labels with SMLM-compatible fluorophores such as Janelia Fluor^{®} fluorophores (for example, Janelia Fluor^{®} 549 and Janelia Fluor^{®} 646) available from Tocris, and tetramethyl rhodamine (TMR).

Optionally, the fluorescent probes are not or do not include a particle (for example, not a quantum dot, nanoparticle).

### Combinations of fluorescent probes

Preferably, the vesicles are labelled with at least two types of fluorescent probe detectable in a different colour channel during the image acquisition step. This requirement may be achieved by choosing fluorescent probes whose emission spectra are positioned at different wavelength bands. To aid resolution of the different fluorophores, the emission spectra of the different fluorophores preferably have regions of relatively low overlap. Differently, and simply, stated - the fluorophores of the fluorescent probes have different emission colours (i.e. produce different colours of fluorescent emission), such that their fluorescence emission can be separated using optical filters (dichroic filters, longpass filters, bandpass filters).

Preferably, at least one of the fluorescent probes is a generic vesicle probe, that is, a probe capable of a labelling a broad spectrum (preferably all) of the vesicles of the test specimen at a relatively high density. Generally, the generic vesicle fluorescent probe is used to label all vesicles, and subsequently imaged by SMLM, so that position data for this fluorescent probe can be used to quickly and accurately derive morphological parameters for each vesicle. To permit this, the generic vesicle fluorescent probe is generally present at relatively high copy number/density. For example, the copy number of the generic vesicle probe may be at least 5 probes per vesicle, preferably at least 10 probes per vesicle, more preferably at least 20 probes per vesicle. Preferred examples of suitable generic vesicle probes include, for example, lectins (WGA or Concanavalin A), a membrane stain (reactive fluorophores, CellMask, Dil, mCLING), a lipid/glycolipid probe (CTxB, annexin V), or a lumen dye.

For example, the first probe may be a green probe and the second probe may be an orange or red probe (the colours referring to the predominant colour of fluorescence emission). In systems using three fluorescent probes, the first probe may be a green probe, the second probe may be an orange probe, and the third probe a red probe. In systems adding a fourth fluorescent probe, this may be in the far red or near-infrared wavelength region. In practice, the fluorescent emission of different probes can overlap to some extent, but the skilled reader is familiar with how to address and (if appropriate) correct for this issue.

Optionally, a first fluorescent probe is imaged by SMLM, and a second fluorescent probe is imaged through diffraction-limited fluorescence microscopy.

In instances where the method of the invention is used to identify a diseased state, the fluorescent probes may include at least one generic fluorescent probe (e.g. a lectin, membrane stain, or a fluorescent species capable of being taken up by the lumen, or one of the other generic vesicle probes described below) and at least one specific fluorescent probe targeting a vesicle biomarker associated with the diseased state. The different fluorescent probes are provided with different colour fluorophores.

For example, in instances in which the invention is used to identify a diseased state, the fluorescent probes may include a generic fluorescent probe such a lectin, and a cancer biomarker such as one of those listed in Table 2 above.

### Vesicle rupture

The vesicles may be left intact prior to imaging - that is, without lysis or rupturing. Alternatively, lysing/rupturing of the vesicles immobilised on the substrate may be carried out prior to imaging to release the vesicles' cargo (the material held in the lumen of the vesicle)-so-called "lysis-based cargo detection". In such instances, at least a portion of the membrane remains immobilised to the substrate after lysis, to facilitate analysis of the vesicle membrane of individual vesicles. It will be understood by the skilled reader that the reference to "vesicles" in the earlier aspects also extends to cover portions of the vesicle immobilised on the substrate, in such embodiments. Lysis may be achieved, for example, through the same chemical and non-chemical permeabilization methods taught above.

In lysis-based cargo detection methods, the substrate preferably includes capture molecules specific to a cargo biomarker, and the labelling step involves labelling the vesicle membrane with one or more fluorescent probes, and labelling said cargo biomarker immobilised on the substrate. In this way, the vesicles can be immobilised and imaged on the substrate surface to build up information about vesicles at an individual level, with population level information about the vesicle cargo on the accessible through analysis of the cargo biomarkers.

In lysis-based cargo detection methods, the sample preparation step preferably comprises:
- providing the surface of the substrate with a first binding agent suitable for capturing vesicles, and a second binding agent suitable for capturing a cargo biomarker;
- immobilising the vesicles on the substrate using said first binding agent;
- lysing the vesicles to release the vesicles' cargo whilst retaining the immobilised vesicles' membrane (or a portion thereof) on the substrate;
- immobilising said cargo biomarker from the vesicles' cargo onto the substrate using said second binding agent;
- (preferably) washing the sample to remove non-bound material; and
- labelling the vesicle membrane and cargo biomarker with at least one fluorescent probe;
- wherein the vesicles are fixed prior to and/or after (preferably immediately prior to and/or immediately after) said labelling step.

The first binding agent and second binding agent may be added to the substrate at the same time, prior to immobilisation of vesicles. Alternatively, the second binding agent may be added after addition of the vesicles, prior to lysing the vesicles.

As described above, the sample preparation step preferably involves passivation of the substrate and/or blocking of the vesicle membrane prior to imaging.

The substrate may be prepared using a method comprising:
i. treating the substrate with a passivation agent to bond the passivation agent to the substrate, wherein at least a fraction of the passivation agent comprises an anchor moiety;
ii. treating the substrate with a mediating compound, the mediating compound having multiple capture moieties suitable for binding to said anchor moiety; and
iii. treating the substrate with a TIM protein (preferably TIM-4), the TIM protein having an anchor moiety which binds to said mediating compound; and
iv. treating the substrate with a cargo biomarker capture molecule to attach the cargo biomarker capture molecule to the substrate (preferably wherein the capture molecule includes said anchor moiety which binds to said mediating compound).

As the skilled reader will understand, steps (iii) and (iv) above can be carried out in any order, or simultaneously. The method may include any of the optional and preferred features set out above.

Following such methods, the substrate may comprise:
- a passivation agent bound to the substrate, at least a fraction of the passivation agent being biotinylated passivation agent;
- multivalent avidin/neutravidin/streptavidin bound to the biotinylated passivation agent; and
- biotinylated TIM protein (preferably TIM-4) bound to the multivalent avidin/neutravidin/streptavidin
- a biotinylated cargo biomarker capture molecule (e.g. an antibody) bound to the multivalent avidin/neutravidin/streptavidin.

### Vesicle-containing sample

Preferably, the test specimen (vesicle-containing sample) is or is derived from a bodily fluid obtained from a human or animal, preferably a human. The bodily fluid may be, for example, saliva, nasal fluid, sweat, breath, urine, semen, cervical mucus, or blood.

The bodily fluid may be purified, for example, by filtering or centrifugation. Preferably, this purification occurs before labelling, to avoid unwanted components of the sample scavenging the fluorescent probes.

Preferably, the sample is filtered prior to any labelling step. Such filtration preferably removes cells which can otherwise become labelled with fluorescent probes and complicate identification of vesicles. Additionally or alternatively, such filtration may remove aggregates, which can otherwise complicate analysis.

Generally, the filtration is carried out with a filter having an average pore size between about 0.2 µm to about 2 µm, more preferably 0.3 µm to 1 µm, more preferably 0.3 to 0.8 µm, most preferably 0.4 µm to 0.5 µm. The filter may be, for example, a nylon membrane filter, such as a Fisherbrand^{™} Nylon Membrane Filter available from Thermo Fisher Scientific.

### Buffer

Suitably, the vesicles of the test specimen are immersed in an imaging buffer.

The imaging buffer may be, for example, phosphate-buffered saline (PBS) or HEPES-buffered saline.

For SMLM imaging by dSTORM, the imaging buffer contains a reducing agent, for example a primary thiol. Suitable primary thiols include, for example β-mercaptoethanol (BME), mercaptoethylamine (MEA), dithiothreitol (DTT) and L-glutathione.

Preferably, in instances where the SMLM is or involves dSTORM, the imaging buffer comprises an oxygen-scavenging system. Suitable oxygen-scavenging systems include, for example, the combination of glucose oxidase and catalase, or the combination of protocatechuic acid (PCA) and protocatechuic dioxygenase (PCD).

Preferably, in instances where the SMLM is or involves dSTORM, the imaging buffer comprises both a reducing agent and oxygen-scavenging system.

In instances where the SMLM is a non-STORM technique, such as PALM, the buffer suitably lacks said reducing agent and oxygen-scavenging system.

For imaging by conventional (non-SMLM) techniques, the imaging buffer preferably lacks a reducing agent and/or oxygen-scavenging system to maximise the number of fluorophores simultaneously.

### Step (2) Image acquisition step

### Single molecule fluorescence imaging

The image acquisition step comprises obtaining image data of individual vesicles.

The image acquisition step includes carrying out fluorescence imaging.

Suitably, the fluorescence imaging includes super-resolution microscopy in which individual fluorophores are imaged and located with sub-diffraction limited accuracy - in other words single molecule localisation microscopy (SMLM). Suitable SMLM techniques include, for example, (Direct) Stochastical Optical Reconstruction Microscopy [(d)STORM], Photo-Activated Localization Microscopy (PALM), Fluorescence-PALM (FPALM), Point Accumulation for Imaging in Nanoscale Topography (PAINT) [in particular, DNA-PAINT], Stimulated Emission Depletion microscopy (STEDM), Ground State Depletion microscopy (GSDM), Spatially Structured Illumination microscopy (SSIM), or combinations thereof. In preferred implementations, the SMLM technique may be dSTORM, PALM, FPALM or PAINT.

STORM, PALM and fPALM rely on building up a picture of the location of photoswitchable fluorophores in a sample by randomly activating a small subset of the photoswitchable fluorophores and determining their precise localisation before the fluorophores either switch back to a dark state or photobleach.

PAINT relies on fluorophores stochastically attaching to a target before detaching/photobleaching. In one implementation, the attaching/detaching is caused through transient binding of a fluorophore-labelled probe to a target. In an especially preferred implementation, the PAINT method is DNA-PAINT, involving transient binding of fluorophore-labelled oligonucleotides to their complementary oligonucleotide on the vesicle. To achieve this in practice, the vesicles may be labelled with a capture probe (as set out above) labelled with a docking oligonucleotide, and treated with fluorophore-labelled imager oligonucletodies which are complementary to the docking oligonucleotide. Details of the DNA-PAINT technique can be found, for example, in Schnitzbauer, J. et al.

In some implementations, the SMLM imaging is 2D-SMLM.

In other implementations, the SMLM is 3D-SMLM, for example 3D PALM or 3D STORM. In such techniques, astigmatism or defocusing is introduced into the imaging system, such that the shape of the detected fluorescence emission from a single molecule can be calibrated with the z position of the molecule. This may be achieved, for example, by introducing a cylindrical lens into the detection optics to create two different focal plans for the x and y directions, so that the shape of the spot detected varies according to the z position of the fluorophore. When fitted with a 2D elliptical Gaussian, as described below, the peak widths in the x and y directions can be related to the z position of the fluorescence emission. Details of such techniques can be found in, for example, Huang *et al.*

### Non-single-molecule fluorescence imaging

Fluorescence imaging may further include carrying out non-SMLM fluorescence microscopy. For example, such a step may involve exciting a bulk population of fluorophores simultaneously, to establish whether a vesicle is positive or negative for a particular biomarker. The overall fluorescence intensity may be used as an indication of the level of fluorophores present, which may be correlated with the amount of biomarker present.

Non-SMLM fluorescence microscopy methods may also include photobleaching step analysis (PBSA), in which the number of fluorophores in a diffraction-limited region is determined based on the number of discrete drops in fluorescence intensity corresponding to independent bleaching events for each fluorophore. This differs from SMLM-based approaches as emission from all fluorophores is inherently simultaneous rather than temporally segregated. As a result, high-precision localisation of individual fluorophores is not possible, however it avoids the inherent stochasticity of SMLM approaches in terms of individual fluorophore detection.

PBSA can be combined with SMLM in the same sample in a number of ways depending on whether the information obtained from each approach is required for the same or different biomarkers. For example, spatial analysis can be performed first using DNA-PAINT SMLM of a biomarker, followed by PBSA of the same biomarker if the transiently binding DNA imaging probes are replaced with a stable hybridisation probe that can ensure a stable 1:1 ratio of target to probe. Alternatively, dSTORM can be performed for biomarkers or a generic stain in one or more channels, and then PBSA performed in the remaining channel(s). In this case, the dSTORM-compatible buffer should be replaced with a PBSA-compatible buffer between acquisition approaches.

In one implementation, the image acquisition step involves non-SMLM imaging of a first fluorescent probe and SMLM imaging of a second fluorescent probe.

### Non-fluorescence imaging

The image data may contain both fluorescence data and data obtained through non-fluorescence imaging methodologies. Suitable non-fluorescence imaging methodologies retain individual vesicle identities, thus allowing both fluorescence and non-fluorescence data to be associated with individual vesicles.

Preferably, the non-fluorescence imaging is a light-based microscopy technique. Advantageously, light-based microscopy may be carried out using the same piece of equipment used to carry out fluorescence imaging. Using the same piece of equipment can simplify assigning both fluorescence and non-fluorescence data to the same vesicles.

As an example of a suitable light-based microscopy are scattering-based imaging of the vesicles, for example, through interferometric scattering microscopy (iSCAT). iSCAT is a label-free method for reporting the mass of molecules or particles through the detection of associated Rayleigh scattering, which is in turn dependent on sample mass and density. For species of a broadly consistent density, such as unmodified EVs, the degree of scattering is determined primarily by the size of the EV.

The size metric as determined by iSCAT can be used in place of, or in combination with, vesicle sizing through a generic fluorescent stain or biomarker-specific SMLM approach. In order to link iSCAT- and SMLM-derived data from the same vesicles, the centroid positions of iSCAT events must be determined and mapped into the same coordinate space as the SMLM localisations. In this way, each iSCAT parameter can be associated with each SMLM cluster, and so each vesicle will be associated with metrics from both imaging sources.

### Imaging protocol

In instances where multiple imaging methods are used, it is preferable for all imaging methods to be applied to a first area of the test specimen, before moving to a second area of the test specimen. This simplifies the registration of results from one imaging method onto those obtained from another imaging method.

Optionally, the method involves drift correction. For example, the method may involve cross-correlation based drift compensation. Alternatively, the drift correction may be based on entropy minimisation, following the approach of Cnossen, J. *et al.* Drift correction can be implemented using the Nanoimager available from Oxford Nanoimaging.

### Fluorescence imaging apparatus

Fluorescence imaging systems suitable for carrying out fluorescence imaging as described above include:
an excitation light source, preferably operable at a range of wavelengths, suitable for exciting the fluorescent probes;
imaging optics (e.g. an objective lens); and
detection optics (e.g. a camera for detecting fluorescence, and preferably optical filters to remove unwanted scatter and background from fluorescence signal).

The excitation light source preferably comprises one or more lasers chosen to match the excitation spectra of the one or more fluorescent probes. In instances where the one or more fluorescent probes comprise fluorophores of different colour, one laser may be capable of exciting multiple (potentially all) of the different fluorophores. More normally, however, the excitation light source comprises multiple lasers which each excite one, or only a subset, of the different fluorophores. For example, the excitation light source may include any combination of a first laser operating below 500 nm (for example, 350 nm-500 nm), a second laser operating between 500-600 nm, a third laser operating between 600-700 nm, and a fourth laser operating above 700 nm. For example, the excitation light source may include lasers operating at 488 nm, 561 nm, 640 nm and/or 750 nm. Preferably, the fluorescence imaging system incorporates lasers capable of emission at three or more wavelengths, optionally four or more wavelengths.

Illumination of the sample may be carried out using any suitable wide-field illumination mode, for example, widefield epifluorescence microscopy (in which the excitation light passes through the objective lens) or light sheet fluorescence microscopy (in which the excitation light source produces a sheet of light illuminated laterally at and parallel to the focal plane of the objective lens) or, more preferably, Total Internal Reflection Fluorescence Microscopy (TIRFM).

Suitably, detection optics include a camera, for example a charge-coupled device (CCD), such as an electron-multiplying CCD (EMCCD), or a complementary metal-oxide semiconductor (CMOS) camera.

The detection optics are preferably capable of detecting and distinguishing multiple colour channels.

To facilitate multi-colour detection, fluorescence emission in different colour channels/bands may be separated and directed to separate pre-determined detector areas.

In one implementation, this is achieved by splitting the emission in different colour channels to separate cameras. However, whilst this retains a large field of view, it results in a relatively bulky and expensive construction, particularly if scaling to 3 or 4 different colour channels.

In another implementation, multi-colour detection is achieved on a single camera by configuring distinct portions of the detector to detect different colour channels. For example, for two-colour channel imaging the emission may be split so that one colour channel is directed to one half of the camera detector, and another camera channel is directed to the other half of the camera detector. For, three or four colour imaging, the camera detector may be split into quarters, in an analogous fashion. The skilled reader is aware of how to achieve this using suitable optical components, and commercially available splitters are available to achieve this configuration, such as the Dual-View^{™} and Quad-View^{™} systems from Optical Insights, LLC. Advantageously, this approach allows simultaneous imaging across multiple colours, and thereby can potentially permit additional information to be obtained by Förster resonance energy transfer (FRET) imaging. In addition, it can permit ratiometric imaging, in which emission from a given fluorescent probe is detectable across multiple colour channels, with a characteristic ratio of fluorescence in the multiple colour channels allowing the fluorescent probe to be identified. To give an example of ratiometric imaging, consider probes A and B, wherein excitation at a wavelength of 488 nm causes probe A to produce fluorescence in colour channel X, and probe B to produce fluorescence in colour channels X and Y, according to an intensity ratio Z. With knowledge of the intensity detected in channel Y and ratio Z, the individual contributions of probes A and B to the signal in channel X can be calculated. This can allow the use of more probes, without increasing overall complexity of the imaging system.

Additionally or alternatively, multi-colour detection is facilitated through the use of a dispersive element as part of the detection optics, such as a prism or grating. The dispersive element can spectrally spread fluorescence emission such that different wavelengths illuminate different parts of a detector. In preferred implementations the dispersive element is a prism. The prism is preferably a compound prism, such as a doublet compound prism. Advantageously, prisms can provide a compact structure for achieving dispersion with a combination of lower photon loss and lower (or no) deviation of emission compared to gratings. Suitably, fluorescent signal is split into at least two detection paths - a first path lacking a dispersive element and a second path having a dispersive element, wherein the first path provides diffraction-limited spots for obtaining position data and the second path provides line spectra for identifying the specific type of fluorophore creating the diffraction-limited spots.

The detection optics may also include a lens for introducing astigmatism to the detected signal, to facilitate 3D-SMLM. For example, the detection optics may incorporate a cylindrical lens.

Suitably, the fluorescence imaging system incorporates a movable stage for mounting the test specimen, for example a motorised stage. Preferably, the stage is controlled by a computer, and the feature vector for each vesicle includes a record of the position of the stage during the image acquisition of that vesicle - e.g. coordinate information defining the position of the vesicle on the test specimen. Suitably, this can allow the system to return to image a specific vesicle as required. For example, if the characterisation step identifies a particular vesicle sub-population, recording coordinate information for each vesicle in that sub-population can allow a user to return to specifically examine or manipulate those vesicles.

### Step (3) Image processing step

In the image processing step, image data obtained from the image acquisition step is processed to obtain information about individual vesicles. The step calculates multiple (at least three) characterising parameters for individual vesicles from position data and/or image data, at least one of the characterising parameters being a morphological parameter, preferably derived from position data for individual fluorescent probes. These characterising parameters are then used to construct an n-dimensional feature vector for each vesicle.

By "characterising parameter" we mean a parameter derived from the image data which reflects a property of the vesicle. Characterising parameters may be a quantitative measure of the size, shape, composition and/or organisation of the vesicle. Additionally or alternatively, characterising parameters may be a qualitative measure of a property of the vesicle e.g. a binary 1 or 0 to reflect the presence or absence of a particular biomarker.

SMLM image data is analysed to acquire position data for individual fluorescent probes. Preferably, a point spread function is fitted to the signal from each fluorescent probe. For example, the point spread function may be a Gaussian function (approximating an Airy disk), and the standard deviation (σ) is recorded as an indication of the location accuracy of the fluorescent probe. Preferably, the signal is fitted with a two-dimensional Gaussian function. Such a Gaussian takes the general form: $f \left(x, y\right) = A exp \left(-\left(\frac{{\left(x-{x}_{o}\right)}^{2}}{2 {σ}_{X}^{2}}+\frac{{\left(y-{y}_{o}\right)}^{2}}{2 {σ}_{Y}^{2}}\right)\right)$ where A is the peak height, x₀ and y₀ are the peak centres, x and y are the spreads about the peak centres, and σ_{X} and σ_{Y} are the standard deviation of the distribution. The standard deviations σ_{X} and σ_{Y} are recorded as two characteristic dimensions of the candidate object. In practice, the axes of the elipse will rarely align perfectly with x and y axes, but instead will be rotated by an angle *Θ*. To account for this, the general form of the Gaussian function fitted to the signal is expressed as: $f \left(x, y\right) = A exp \left(-\left(a{\left(x-{x}_{o}\right)}^{2}+2b\left(x-{x}_{o}\right)\left(y-{y}_{o}\right)+c{\left(y-{y}_{o}\right)}^{2}\right)\right)$ in which: $a = \frac{{cos}^{2} θ}{2 {σ}_{X}^{2}} + \frac{{sin}^{2} θ}{2 {σ}_{Y}^{2}}$ $b = - \frac{sin 2 θ}{4 {σ}_{X}^{2}} + \frac{sin 2 θ}{4 {σ}_{Y}^{2}}$ $c = \frac{{sin}^{2} θ}{2 {σ}_{X}^{2}} + \frac{{cos}^{2} θ}{2 {σ}_{Y}^{2}}$ and the matrix: $\left[\begin{matrix}a & b \\ b & c\end{matrix}\right]$ is positive-definite.

As noted above, in instances where 3D-SMLM is carried out, the σ_{X} and σ_{Y} values can be used to provide information about the z-position of the fluorescent probe.

Optionally, the position data is filtered by quality metrics. For example, the position data for a given fluorescent probe may be rejected in instances where the photon count is below a given threshold, where the standard deviation of the fitted point spread function is above a given threshold, or where the fitted point spread function does not satisfy a suitable goodness of fit test.

In most instances, a given fluorescent probe will be visible across multiple frames of a video image of the vesicle. In such instances, calculating position data may correspond to calculating averaged position data for a fluorescent probe across multiple (optionally all) video frames. This may be implemented, for example, by identifying fluorescent signal occurring in the same region across adjacent frames. For example, calculating averaged position data may comprise identifying fluorescent signal occurring within a region no more than 200 nm in diameter, no more than 100 nm in diameter, no more than 80 nm in diameter, no more than 60 nm in diameter, or no more than 40 nm in diameter across multiple frames. Optionally, calculation of the averaged position data allows for gap frames (e.g. 1 frame, 2 frames or 3 frames) in which fluorescent signal temporarily disappears within said region before reappearing, which can arise due to the fluorescent probe temporarily entering a dark state. The averaged position data may correspond to an average value (e.g. mean or medium) for the position, photon count, standard deviation of the fit, goodness of fit and so on. Optionally, the averaged position data is rejected if the fluorescent signal remains visible above a threshold number of frames, since excessively long visibility could be indicative of imaging of an impurity or fluorophores which are not photobleaching or entering a dark state as expected. The threshold number of frames may be, for example, 10 frames, 12 frames, 15 frames, or 20 frames.

The image processing step comprises associating the position data for individual fluorophores with individual vesicles.

Associating position data with individual vesicles may be carried out by any suitable technique.

For example, associating position data with individual vesicles may be carried out using a clustering algorithm. The clustering algorithm may be, for example, an HDBSCAN clustering algorithm as described, for example, in Campello et al, as well as on the following web page: https://hdbscan.readthedocs.io/en/latest/how_hdbscan_works.html. Alternatively, the clustering algorithm may employ Voronoi tessellation, as taught in Lee *et al.* Suitably, the clustering algorithm is constrained to limit or prevent false clustering of data, for example, by constraining acceptable circularity and diameter values to specific ranges. For example, the clustering algorithm may require a circularity of 0.8-1 and diameter of 40-500 nm. Such constraints can be particularly important when the fluorescent probes are clustered on the vesicle surface, for example, through localisation of the fluorescent probes to specific domains on the vesicle.

Alternatively, associating position data with individual vesicles may involve identifying regions of the image with a localisation density above a given threshold.

Optionally, associating position data with individual vesicles may involve identifying ring-shaped accumulations of individual fluorescent probes. The ring-shaped accumulations may be indicative of the membrane of a vesicle.

In instances where SMLM image data is obtained in multiple colour channels, the position data in the multiple colour channels may be combined/pooled. Pooling position data in this way can help to increase the accuracy of cluster identification.

Associating position data may also be carried out by comparing position data from SMLM image data against image data obtained through a non-SMLM technique - for example, by comparing position data against a non-SMLM image of the same vesicles or an iSCAT image of the same vesicles.

Optionally, associating position data with individual vesicles may be carried out through a combination of clustering analysis of SMLM data and comparison with image data of the same vesicles obtained through a non-SMLM technique.

The position data for individual fluorophores associated with a given vesicle is then used to calculate characterising parameters for that vesicle.

The characterising parameters include at least one morphological parameter. A "morphological parameter" is a parameter which characterises the shape or size of the vesicle.

Optionally, the at least one morphological parameter includes a perimeter of the vesicle. When obtained through SMLM data, the perimeter of the vesicle may be obtained by connecting the positions of the outermost fluorophore positions associated with an individual vesicle, and measuring the overall length of the connections between those positions. Optionally, these positions may be fitted with a spline, such as a smoothing spline, with the perimeter determined to be the length of the spline.

Optionally, the at least one morphological parameter includes a diameter of the vesicle. The diameter of the vesicle may be determined based on the smallest circle incorporating all of the fluorophore positions associated with a vesicle. Alternatively, the diameter of the vesicle may be calculated based on a circle having the same circumference as the measured perimeter of the vesicle or the same area as that encompassed by the perimeter of the vesicle. Alternatively, the diameter of the vesicle is calculated by fitting a circle to the perimeter.

Optionally, the at least one morphological parameter includes the circularity of the vesicle. The circularity of the vesicle may be obtained by determining the perimeter of the vesicle in the manner taught above and then calculating the deviation from a perfect circle.

Optionally, the morphological parameter is derived from said position data for individual fluorescent probes by identifying ring-shaped accumulations of individual fluorescent probes indicative of the membrane of a vesicle.

Optionally, the at least one morphological parameter includes at least one measure of the area of the vesicle. For example, the area may be based on the area encompassed by the perimeter of the vesicle as discussed above. Alternatively, the area may be calculated based on the diameter of the vesicle, as taught above.

Optionally, the at least one morphological parameter includes the skew of the vesicle.

Preferably, the at least one morphological parameter includes at least a perimeter parameter, a diameter parameter, and an area parameter. More preferably, the at least one morphological parameter includes at least a perimeter parameter, a diameter parameter, an area parameter, a circularity parameter and optionally a skew parameter. Advantageously, SMLM-data allows all of these measures to be calculated in a straightforward way.

Preferably, the characterising parameters include at least one distribution parameter related to the position data of the fluorescent probes. A "distribution parameter" is a measure of the positions of the fluorescent probes relative to one another and/or to the vesicle itself.

The distribution parameter may include nearest-neighbour distance, extent of surface coverage, and/or clusteredness in a single colour channel.

In preferred implementations in which multicolour imaging of multiple fluorescent probes is employed, the characterising parameters preferably include at least one co-localisation parameter. A "co-localisation parameter" is a measure of whether different species are close/associated on the vesicle. The co-localisation parameter may be any or all of nearest neighbour between molecules of different colour, colocalization between biomarkers of different colour, ratio of relative biomarker abundance.

Dimensionality can be further increased through the use of higher-order functions or complex spatial analyses such as Ripley's K or Pair Correlation Function. In such an approach, the first-order function would describe overall biomarker density, the second-order function would describe clustering (e.g. Ripley's K), and the third-order would describe bandedness or equivalent matrix-derived features. A further example of spatial analysis may involve spatial frequency-based distribution, which may be calculated from spatial Fourier transforms, especially those that can be attributed to a 2D or 3D surface.

Optionally, the characterising parameters for individual vesicles include at least one parameter derived for the vesicle from non-SMLM image data.

For example, the characterising parameters may include a morphological parameter determined by iSCAT (e.g. vesicle size). In addition, or alternatively, the characterising parameters may include parameters derived from non-SMLM fluorescent imaging, such as a copy number parameter determined by PBSA and/or a binary "present" or "not present" parameter related to the presence of a particular biomarker.

In instances where the characterising parameters for a given vesicle are derived from both SMLM image data and non-SMLM image data, the image processing step may involve an image registration step. This ensures that the SMLM image data and non-SMLM image data both relate to the same individual vesicle.

For example, where the non-SMLM image data is iSCAT data, image registration may comprise calculating the centroid position of iSCAT events and mapping onto the same coordinate space as the SMLM image data.

Similarly, where the non-SMLM image data includes non-SMLM fluorescent images, the image registration may comprise determining the central position of a fluorescent object (e.g. through determining the centroid position of an object or fitting to a suitable profile e.g. a 2D Gaussian as taught above) and mapping onto the same coordinate space as the SMLM image data.

### Feature vector

The image processing step involves constructing a feature vector for individual vesicles. The feature vector comprises all of the characterising parameters associated with that vesicle. It is an n-dimensional vector, where each of the characterising parameters corresponds to one of the n-dimensions.

The feature vector for an individual vesicle includes at least 3 characterising parameters, preferably at least 4, preferably at least 5, preferably at least 6, preferably at 7, preferably at least 8, preferably at least 9, preferably at least 10.

### Step (4) Data transformation step

The feature vectors resulting from the image processing step have at least 3 dimensions, and preferably have far more to maximise the amount of information about each vesicle. However, this type of high-dimension data can be hard to process and interpret. Thus, a key feature of the present invention is that the feature vectors are subjected to a data transformation step, to reduce the number of dimensions to a more manageable level whilst minimising loss of information.

This is achieved by inputting the n-dimensional feature vectors into a dimensionality reduction algorithm to form a modified m-dimensional feature vector, where m is less than n.

Preferably, the modified feature vectors are two- or three-dimensional (i.e. m = 2 or 3), preferably two-dimensional.

Preferably, the dimensionality reduction algorithm is or includes t-Distributed Stochastic Neighbour Embedding (t-SNE).

Optionally, the dimensionality reduction algorithm is or includes a principal component analysis ("PCA") algorithm. This converts the original feature vector into a modified feature vector based on linear combinations of the original features. Details of how to carry out PCA can be found, for example, in Joliffe and Cadima.

Optionally, the dimensionality reduction algorithm is or includes a uniform manifold approximation and projection ("UMAP") algorithm.

Optionally, the dimensionality reduction algorithm is a multi-step process, for example, sequentially deploying the algorithms mentioned above. For example, the dimensionality reduction algorithm may first apply a principal component analysis to reduce the n-dimensional feature vector to an o-dimensional feature vector, before applying UMAP or tSNE to reduce the o-dimensional feature vector to said m-dimensionsal feature vector.

The data transformation step may also facilitate distinguishing between different types of vesicles. This may be achieved in the data transformation step by weighting the different characterising parameters, or choosing a perplexity value for t-SNE, which results in the modified feature vectors for different types of vesicles being separated (e.g. maximally separated) in modified feature vector space.

For example, where a user wants to distinguish between a first type of vesicle and a second type of vesicle, the data transformation step may be configured to produce modified feature vectors that that are separated (e.g. extend in different directions in modified feature vector space) for the two types of vesicles. This may be achieved in the data transformation step, for example, by weighting the characterising parameters in the feature vector based on known properties of the first and second types of vesicles. Then, where a modified feature vector corresponds to the first type of vesicle, the modified feature vector may extend in a first direction in modified feature vector space. Where a modified feature vector corresponds to the second type of vesicle, the modified feature vector may extend in a second direction in modified feature vector space. This may facilitate distinguishing between the first type and the second type of vesicle.

In some cases, the system may comprise a set of predetermined protocols for performing the data transformation step. Each protocol may correspond to one or more vesicle types which the user wants to distinguish or characterise. Each protocol may include a set of predetermined parameters (e.g. weighting parameters, and/or a perplexity value for t-SNE) for the data transformation step, which are configured to produce modified feature vectors which facilitate detecting or distinguishing the one or more vesicle types of interest. The predetermined parameters may be determined, for example, based on known properties (e.g. properties corresponding to the characterising parameters included in the feature vector) of the one or more vesicle types.

### Step (5) Characterisation step

In the characterisation step, the vesicles are characterised/classified. This is achieved by comparing the modified feature vector of that vesicle with internal reference data (other modified features obtained for the same test specimen) or external reference data (obtained from other samples, or through modelling).

The characterisation step may involve assigning identified vesicles into two or more sub-populations of vesicles. The number of sub-populations may be, for example, at least two, at least three, at least four, or at least five.

Assigning identified vesicles into two or more sub-populations may comprise carrying out clustering analysis of the modified feature vectors. For example, the clustering analysis may involve use of graph-based clustering (such as a k-nearest neighbour analysis, (for example, the weighted-nearest neighbour analysis implemented in Seurat version 4, available from the Satija Lab at https://satijalab.org/seurat/index.html), k means clustering, or HDBSCAN. Examples of suitable clustering methodologies are described in Andrews and Hemberg et al., available at *https:*//*www.sciencedirect.com*/*science*/*article*/*pii*/*S0098299717300493.* Advantageously, carrying out dimensionality reduction before this clustering analysis can decrease the computational complexity.

Characterisation of the cells relative to external reference data may be carried out using a machine learning classification algorithm, such as logistic regression or a convolutional neural network (CNN). The classification algorithm can be created by fitting a training dataset using machine learning analysis, to link characterising parameters to defined classification types. A supervised learning algorithm may be used to fit the training dataset, e.g. a logistic regression algorithm. Details of such approaches are described, for example, in Deep Learning by lan Goodfellow, Yoshua Bengio, and Aaron Courville (MIT Press, 2016), in particular section 5.7. The logistic regression algorithm may be implemented using the MatLab software package, for example by using the Machine Learning and Deep Learning application package to train the system, and analysing sample data using the mnrfit function (as described, for example at https://uk.mathworks.com/help/stats/train-logistic-regression-classifiers-in-classification-learner-app.html and https://uk.mathworks.com/help/stats/mnrfit.html).

### Step (6A) Diagnostic step

Optionally, the results from the characterisation step are used in a diagnostic step. In the diagnostic step, a particular clinical picture is formed form the results.

The output from the characterisation step may be used to identify a disease state. This may occur, for example, through the presence of a particular type of biomarker in the vesicles, or particular population characteristics for the vesicles (e.g. size or morphology).

In other words, the present invention may provide a method of diagnosing a disease in a patient comprising testing a sample of vesicles obtained from the patient in the methods as set out above, wherein the characterisation step involves comparing the modified feature vector for vesicles against reference data obtained from patients confirmed to possess the disease.

The disease may be, for example, cancer. In such instances, the one or more fluorescent probes may include a generic vesicle probe and a fluorescent-labelled capture molecule targeting a cancer biomarker. The cancer biomarker may be any of those set out above including, for example, EGFR or PD-L1.

Additionally or alternatively, the output from the characterisation step may be used to identify the physiological origin of the vesicles. For example, the characterisation step may be used to identify which tissue the vesicles arise from.

### Step (6B) Sub-population analysis

In instances where the characterisation step involves assigning EVs into two or more sub-populations, individual sub-populations may be subjected to further data analysis.

For example, the method may involve averaging position data and/or characterising parameters across the sub-population.

The method may involve creating a composite image representing the spatial features of the sub-population of vesicles.

Creating a composite image may involve combining the position data from multiple vesicles from the same sub-population. Generally, the position data from each vesicle is normalised and aligned before being combined. Normalising may involve scaling the position data of an vesicle based on a morphological parameter of that vesicle (in particular, the diameter). Aligning the data may involve axially aligning each vesicle, by aligning the centroid of each vesicle. Aligning the data may also involve radially aligning each vesicle, for example, identifying the peak density of a given biomarker around the outer perimeter (the region of the outer perimeter of an vesicle with the greatest density of a given biomarker), forming a meridian by connecting this region to the centroid of the vesicle, and then aligning the meridians of each vesicle. In instances where the position data includes data for two different biomarkers, the first biomarker may be used to determine the radial alignment, and a second biomarker may be used for further alignment - for example, if the peak density of the first biomarker is used to achieve radial alignment, the peak density of the second biomarker may be set so as to always appear in the clockwise direction (if necessary through inverting the position data about a mirror plan along said meridian - possible due to the symmetry of vesicle shape).

Once normalised and aligned, the composite image may be obtained by summing the position data across each of the vesicles. Alternatively, the composite image may be an averaged image.

The same system may also be extended to 3-dimensional position data obtained by 3D-SMLM - normalising the position data and aligning the position data based on peak density.

The composite image can be used to help differentiate sub-populations. For example, the composite image may provide a simple visualisation allowing sub-populations to be distinguished from one another.

Averaging position data and/or characterising parameters can be useful for refining the analysis methods used in the invention. For example, if it is discovered that one characterising parameter in particular shows minimal variation between all vesicles of the sub-population (e.g. with a low standard deviation) then the weighting assigned to such characterising parameters may be increased in the dimensionality reduction analysis.

In addition, or alternatively, after identifying sub-populations using the modified feature vectors, the method may involve analysing the characterising parameters for a given sub-population. For example, once a particular sub-population has been identified, the analysis may look for correlations between characterising parameters of that sub-population - for example, whether the copy number of a particular fluorescent probe correlates with vesicle size, or vesicle skew correlates with vesicle size for that sub-population.

### Step (6C) Sub-population targeting

In instances where the characterisation step involves assigning vesicles into two or more sub-populations, individual sub-populations may be selected for further examination.

For example, in instances where a specific vesicle sub-population has been identified, the method may involve manipulating those specific vesicles. For example, the method may involve laser ablating the specific vesicles and collecting the material for downstream mass spectrometry.

Alternatively, the method may involve specifically cleaving certain populations of vesicles from the surface. For example, the vesicles may be attached to the substrate by a photocleavable moiety, allowing the vesicles to be liberated from the substrate upon irradiation with light of a suitable wavelength whilst the vesicles remain intact. Examples of suitable photocleavable moieties include, for example, 2-nitrobenzyl, which undergoes photocleavage upon UV irradiation. Suitable photocleavable linkers are commercially available, such as PC biotin-PEG3-NHS ester available from Sigma-Aldrich, having the following formula:

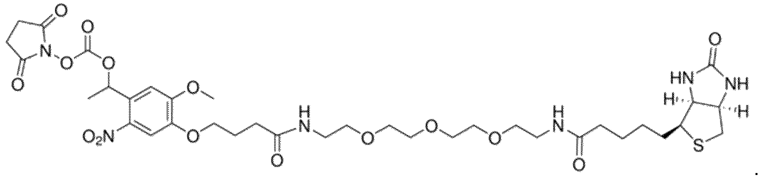

In some implementations, the liberated vesicles are collected. This can allow a "pure" sample of the specific sub-population of vesicles to be obtained. This can allow the sub-population to be subjected to further tests.

Alternatively, or additionally, after removal of the specific sub-population of vesicles the remaining substrate-bound vesicles may be subjected to further analysis.

### Specific practical applications

As noted above, potential uses for the method of the present invention include the identification of tissue origin of a sample of vesicles. Using a focussed staining panel that affords maximal discrimination between vesicles released by different cell types, vesicles from different tissues can be clustered according to biomarker profile and other vesicle parameters. This can be achieved by comparing the modified feature vector for vesicles against modified feature vectors obtained from reference data relating to vesicles from a known tissue origin. This comparison can be achieved, for example, through training a machine learning algorithm based on reference data from vesicles of known tissue origin, and then using the machine learning algorithm to characterise the vesicles of the test specimen.

For example, vesicles released from mature dendritic cells are enriched for proteins such as class II MHC, ICAM1, and B7 proteins (CD80/CD86), which are required for their role in costimulatory function to T cells, whereas vesicles derived from T cells themselves are enriched for alternative markers such as TCR, CD4/8, CD69.

Such identification of tissue origin allows the specific interrogation of vesicle profiles from a given tissue/organ in order to examine function or diagnose dysfunction. Examples include:
1. An increased number of apoptotic bodies versus exosomes/microvesicles in vesicles from a given tissue indicating damage to that tissue. The nature of the vesicles derived from a given tissue can be determined by reference to vesicle size (reported by iSCAT or SMLM), since apoptotic bodies are substantially larger (>1 µm) than exosomes/microvesicles (10s-100s nm).

Thus, in a further aspect the present invention provides a method of diagnosing tissue damage, comprising:
(1) a sample preparation step, comprising obtaining a sample of vesicles from a tissue, labelling the vesicles with one or more fluorescent probes, and preparing a test specimen by attaching said vesicles to a substrate;
(2) an image acquisition step, comprising imaging said one or more fluorescent probes on the vesicles to generate image data ( using single molecule localisation microscopy (SMLM) of said one or more fluorescent probes to generate image data including SMLM image data);
(3) an image processing step, comprising:
   Identifying individual vesicles in the image data,
      calculating at least three characterising parameters for individual vesicles from the image data, at least one of the characterising parameters being a morphological parameter, and
   constructing a feature vector for individual vesicles from the characterising parameters;
(4) a data transformation step, comprising inputting the feature vectors for individual vesicles into a dimensionality reduction algorithm to calculate modified feature vectors of lower dimensionality for individual vesicles;
(5) a characterisation step, involving characterising each vesicle by comparing the modified feature vector for that vesicle against reference data, to identify which of the vesicles are exosomes/microsomes and which are apoptotic bodies; and
(6) a diagnosis step, comprising diagnosing tissue damage if the proportion of apoptotic bodies is above a threshold amount.

2. The expression of stress/cancer markers on vesicles from a particular tissue, indicating dysfunction. The majority of such markers are not inherently markers of dysfunction and so understanding their tissue context is essential to gaining usable diagnostic information. For example, elevated EGFR on vesicles derived from pancreatic or gastric tissue may indicate the presence of cancer in those tissues. Another example is the immunoinhibitory ligand PDL1, which is legitimately expressed in cells of lymphoid and myeloid lineages, but is a marker of cancer in e.g. melanocytes. The precise quantification of the abundance of such markers on cancer vesicles (e.g. using PBSA) may be highly relevant in many situations where minor differences in biomarker abundance has a physiological significance.

3. Differential abundance of vesicles from different tissues across individuals or conditions, and the potential for correlation with medical conditions and/or response to therapy.

4. The identification of target tissues in the event of viral infection. This has research utility when examining the infectiousness of a new virus, but also has diagnostic implications since understanding the identity of primarily infected tissues in a given patient informs treatment. For example, SARS-CoV2 is able to infect a broad range of tissues, including the upper and lower respiratory tracts, salivary glands, liver, and kidneys; and understanding which is the main site of infection in a patient would allow clinicians to deliver the most appropriate treatment. The detection of viral biomarkers (which frequently integrate into vesicles due to the shared routes of vesicle and virus biogenesis) on vesicles of a given tissue origin would provide this information. The incorporation of particle averaging as part of this analysis may inform understanding of the nature of infection in different tissues - e.g. if vesicles derived from two separate tissues both contain viral antigen but in one it has a distribution more similar to that in whole virions then it may indicate that viral burden and/or mechanism of processing differs between the two tissues.

Importantly, the uses above are all compatible with non-invasive collection of vesicles, such as from patient serum. Serum-derived vesicles are known to report the conditions within solid tissues (e.g. Li, C. *et al*), and so profiling vesicles from serum or other biofluids is in effect a screening tool for all tissues exposed to that biofluid. This represents whole-body clinical profiling for screening a wide range of conditions without the need for prior indications.

Also as noted above, the methods of the present invention also have applicability to analysis of immune cells both for diagnostic and therapeutic purposes.

For example, the present invention also includes a method of identifying and characterising vesicles originating from immune cells using a method of the first aspect of the invention, wherein the one or more fluorescent probes include probes specific to cell-specific biomarkers (e.g. CD4, CD8, TCR, BCR). The method may be used, for example, to characterise the relevant prevalence of populations of CD4+ and CD8+ vesicles (using CD4 and CD8 specific fluorescent probes), and the characteristics of said vesicles. The method may also be used for characterising vesicles derived from cells used in adoptive cell therapy, such as CAR-T therapy, for example by using fluorescent probes specific to a characteristic biomarker (e.g. CAR).

### BRIEF DESCRIPTION OF THE DRAWINGS

Embodiments and experiments illustrating the principles of the invention will now be discussed with reference to the accompanying figures in which:
Figure 1 is a flowchart which illustrates a method of imaging and characterising vesicles according to the invention;
Figures 2A-2D are schematics showing the production of a preferred substrate for immobilising vesicles during imaging;
Figure 3 is a schematic showing a partial cross-section of a vesicle bound to the substrate of Figure 2D;
Figures 4A-4C are schematics showing the type of information obtained and used in the method of the invention - specifically, single molecule localisations of a high density generic vesicle stain (Figure 4A), single molecule localisations of a biomarker-specific fluorescent probe (Figure 4B), and a perimeter of the vesicle obtained from detected locations of the high density generic vesicle stain;
Figure 5 is a schematic of different vesicles smaller than the diffraction limit of visible light, which shows the ability of the method of the present invention to distinguish between different types of vesicle;
Figure 6 is a schematic of equipment suitable for carrying out a method of the invention.
Figures 7A-7J are plots showing simulated data for characteristic features of 5 different populations of vesicles, S1-S5.
Figure 8 shows the results of principal component analysis carried out on S1-S5; and
Figure 9 shows the results of UMAP analysis carried out on S1-S5.

### DETAILED DESCRIPTION

Aspects and embodiments of the present invention will now be discussed with reference to the accompanying figures. Further aspects and embodiments will be apparent to those skilled in the art.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

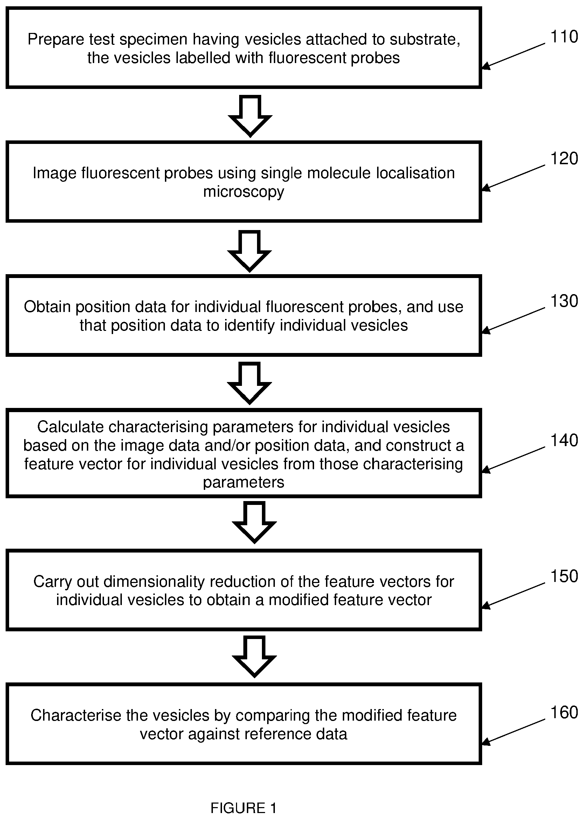

Figure 1 is a flowchart that illustrates a method for characterising vesicles according to the invention.

At step 110, a test specimen is prepared by immobilising vesicles onto a substrate. The vesicles are labelled with fluorescent probes, before and/or after immobilisation. In step 120, the fluorescent probes are imaged using SMLM. This is followed, in step 130, by using position data obtained through SMLM to identify individual vesicles, for example, through using a clustering algorithm such as HDBSCAN. Once individual vesicles have been identified, characterising parameters are then calculated for individual vesicles in step 140, based on the position data and/or image data. The characterising parameters include at least one morphological parameter, such as the diameter. A feature vector is then constructed for each individual vesicle, which includes the various characterising parameters and, optionally, identifying information such as the coordinates of the vesicle on the microscope sample stage. In step 150, the feature vector is subjected to dimensionality reduction, to make the size of the feature vector more manageable for future analysis and interpretation. Then, in step 160, the individual vesicles are characterised through a comparison of the modified feature vector with a suitable reference, whether that be an internal reference from within the same sample or an external reference from a different reference sample.

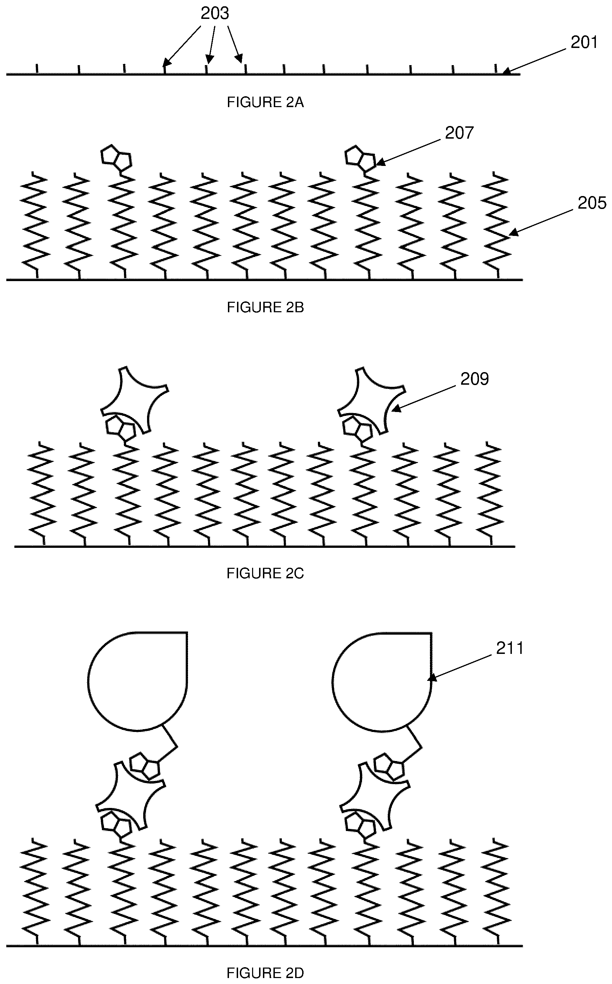

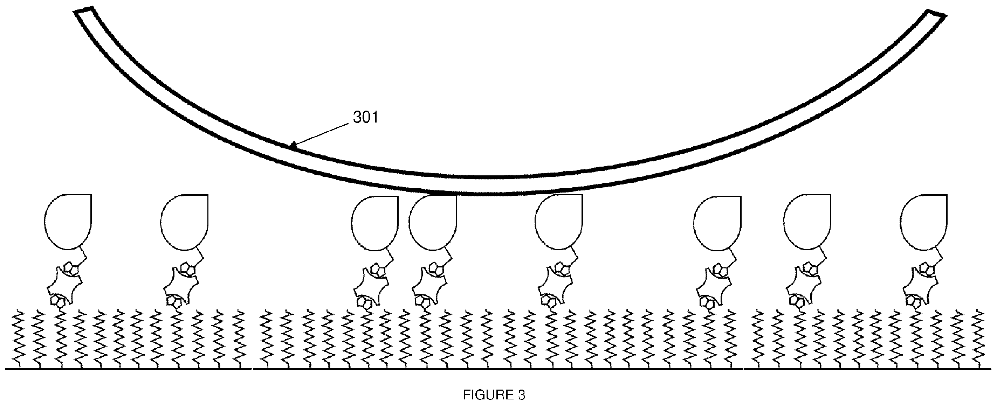

Figures 2A-2D and Figure 3 show a preferred implementation for immobilising vesicles in step 110. In Figure 2A a glass slide 201 has been cleaned using piranha solution, with functional groups 203 added. The functional groups 203 are provided through reaction of hydroxyl groups on the glass slide 201 with 3-aminopropyltriethoxysilane. In Figure 2B, the glass slide has been treated with a mixture of PEG 205 and biotinylated PEG 207, both of which have reacted with the functional groups 203 on the glass slide so as to become covalently bonded to the slide. Next, neutravidin 209 is added as shown in Figure 2C, before addition of biotinylated TIM-4 protein 211 in Figure 2D.

Figure 3 shows the slide of Figure 2D after addition of a vesicle 301 to the surface. Phosphatidylserine in the membrane of the vesicle 301 has become attached to the TIM-4 protein 211, thereby immobilising the vesicle. The density of the TIM-4 is such that the vesicle is secured through interaction with multiple TIM-4 proteins.

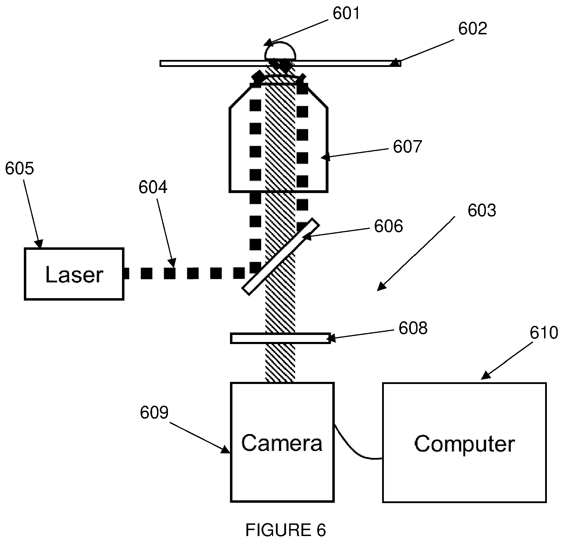

A super-resolution fluorescence microscopy system suitable for carrying out step 120 is shown in Figure 6. Figure 6 shows a test specimen 601 mounted on motorised stage 602. The test specimen consists of a plurality of vesicles immobilised on a coverslip and immersed in an imaging buffer. The imaging buffer is compatible with dSTORM, containing a reducing agent (e.g. a primary thiol such as β-mercaptoethanol (BME), mercaptoethylamine (MEA), dithiothreitol (DTT) or L-glutathione) and an oxygen scavenging system (e.g. the combination of glucose oxidase and catalase, or the combination of protocatechuic acid (PCA) and protocatechuic dioxygenase (PCD)). The vesicles have been labelled with a dSTORM compatible fluorescent probe having specificity to a biomarker on the vesicle surface, and have been fixed prior to imaging to preserve clustering information. The dSTORM compatible fluorescent probe includes a photoswitchable fluorophore, which is able to switch from a dark state to an emissive state.

The sample 601 is interrogated by Total Internal Reflection Fluorescence Microscopy (TIRFM) system 603. In the TIRFM system 603, excitation beam 604 from laser 605 is reflected by dichroic mirror 606 so as to pass through the edge of objective lens 607, and totally internally reflect off the top surface of coverslip. This creates an evanescent field, which switches a small proportion of the photoswitchable fluorescent probes from a dark to an emissive state. Fluorescence emission from the emissive fluorescent probes is then collected by objective lens 607 and passes through dichroic mirror 606 and optical filter 608 before being detected on EMCCD camera 609. Signal from the emissive fluorescent probes then disappears, either due to the fluorophore switching back to a dark state or photobleaching. Through control of conditions (in particular laser power), the density of photoactivated fluorescent markers in each image recorded by the camera is such as to allow individual fluorescent markers to be identified as separate points. By acquiring multiple images, it is possible to gradually construct an image of individual fluorescent markers across the cell surface.

Data from EMCCD is fed to computer 610 for storage and processing. Computer 610 is configured to carry out the remaining steps of Figure 1.

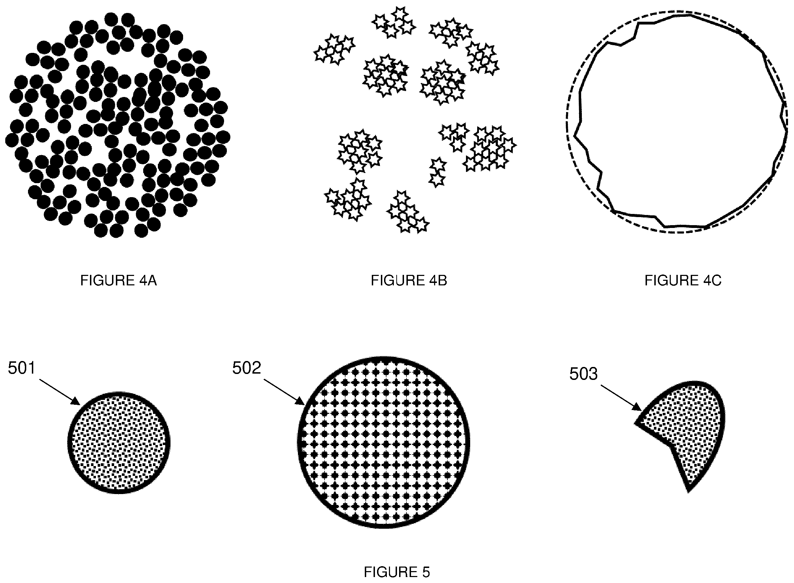

Figures 4A-4C show data obtained for an individual vesicle after step 120.

Figure 4A shows position data for a biomarker "A" widely present on the surface of the vesicle, in this case, WGA fluorescently labelled with a single dye molecule. The WGA has been imaged using dSTORM, and the fluorescent signal from the markers fitted with a 2D Gaussian function. The black circles shown in Figure 4A are centred at the peak of each Gaussian with the circle radius corresponding to the standard deviation of the fit (generally taken to be a measure of the localization accuracy), in this case corresponding to around 10 nm. From this, it can be seen that the overall size of the vesicle is below the diffraction limit of visible light.

Although not shown in Figure 4A, signal from the WGA may appear more concentrated around the perimeter of the vesicle since the area of membrane associated with each pixel generally increases away from the centre due to curvature of the membrane, resulting in a greater number of biomarkers per pixel towards the perimeter of the vesicle. In such instances, this can lead to ring-shaped accumulation of signals from the WGA. These ring-shaped structures can be used to identify individual vesicles within the membrane, and distinguish from non-intact vesicles, membrane fragments, or other contaminants.

Figure 4B shows position data for another biomarker "B" which is present at a lower copy number compared to that in Figure 4A. From the raw image, it is clear that the biomarker is clustered on the vesicle surface.

In this instance, individual vesicles have been located based on biomarker "A", due to its greater prevalence and its lower propensity to cluster (which might otherwise lead to erroneous identification of a cluster as a separate vesicle).

Finally, in Figure 4C the position data in Figure 4A has been used to estimate the size of the vesicle. The process begins by identifying the centroids of the outermost circles in Figure 4A. An estimate of the perimeter of the vesicle can be obtained by linking together adjacent centroids, as shown in the solid line of Figure 4C. Alternatively, the perimeter may be estimated based on the smallest circle which encompass all the outermost points of Figure 4A (on the assumption that the vesicle will be spherical), represented by the dashed line.

In this instance, based on the data in Figures 4A-4C, the method involves constructing a feature vector containing the copy number of biomarkers A and B, the number and size of clusters of biomarker A, the number and size of clusters of biomarker B, the nearest neighbour distance between biomarkers A and B. The feature vector also includes the diameter of the vesicle, based on the dashed line of Figure 4C.

Figure 5 is intended to illustrate the advantages of SMLM analysis according to the invention compared to conventional diffraction-limited fluorescence microscopy. The figure shows intact vesicles 501 and 502, and a damaged vesicle 503 which are all less than 150 nm in diameter, and all labelled with a fluorescent probe represented by the black dots. Vesicles 501, 502 and 503 all include the same copy number of the fluorescent probe. Using an SMLM method in accordance with the invention, vesicle 501 can be distinguished from 502 due to its smaller size, in spite of having the same copy number of fluorescent probes. Similarly, the method can distinguish vesicles 501 and 502 from vesicle 503 due to the different shape of vesicle 503, and can identify that vesicle 503 is damaged due to its non-spherical shape. In contrast, under conventional diffraction-limited fluorescence microscopy the signal from each vesicle 501, 502, 503 would appear identical.

### EXAMPLE

To demonstrate the ability of the method of the invention to classify vesicles, the method was tested using simulated data.

The simulated data contained five different populations of vesicles, referred to as S1 to S5, all labelled with fluorescent probes A-C. A "low", "medium" or "high" level designation was set against ten different characterising parameters for each of S1 to S5 - vesicle size (i.e. a morphological parameter), the copy number of probes A-C, the clusteredness of probes A-C, and the distance between probes of different types (a measure of colocalization). These data were representative of the types of information accessible through use of SMLM, according to the method of the invention. A mean and standard deviation value were set for each level of each characterising parameter. Feature vectors were then simulated for individual vesicles in each of S1-S5 by populating the vector with characterising values based on the mean associated with the appropriate level, with random variability introduced based on the standard deviation value. Approximately 400 vesicles were simulated for each of S1 to S5.

**Table 3**

| | **S1** | **S2** | **S3** | **S4** | **S5** | **Biological interpretation** |
|---|---|---|---|---|---|---|
| **Numbers A** | M | L | M | M | M | On average S2 has approx 50% of the total number of A molecules/EV compared to the other populations |
| **Numbers B** | M | L | M | M | M | On average S2 has approx 50% of the total number of B molecules/EV compared to the other populations |
| **Numbers C** | M | M | L | L | M | On average S3 and S4 have approx 50% of the total number of C molecules/EV compared to the other populations |
| **Cluster density A** | L | L | M | M | L | S1, S2, and S5 show lower density clustering of biomarker A than S3 and S4 |
| **Cluster density B** | L | L | M | M | M | S1 and S2 show lower density clustering of biomarker B than S3, S4, and S5 |
| **Cluster density C** | L | L | L | L | M | S1 to S4 show lower density clustering of biomarker C than S5 |
| **A-B distance** | L | M | M | M | M | Clusters of biomarkers A and B are more closely co-localised in S4 than the other populations |
| **A-C distance** | M | M | M | M | M | All populations show equivalent co-localisation of clusters of biomarkers A and C |
| **B-C distance** | M | M | M | M | L | Clusters of biomarkers Band C are more closely co-localised in S5 than the other populations |
| **Diameter** | M | M | L | M | H | S3 EVs are on average smaller than the other populations, and S5 are on average larger |

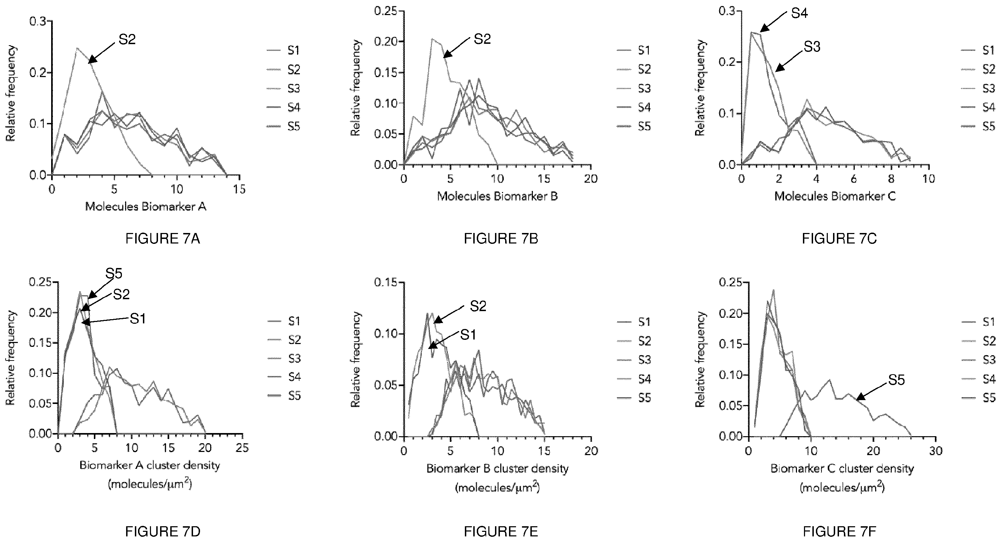

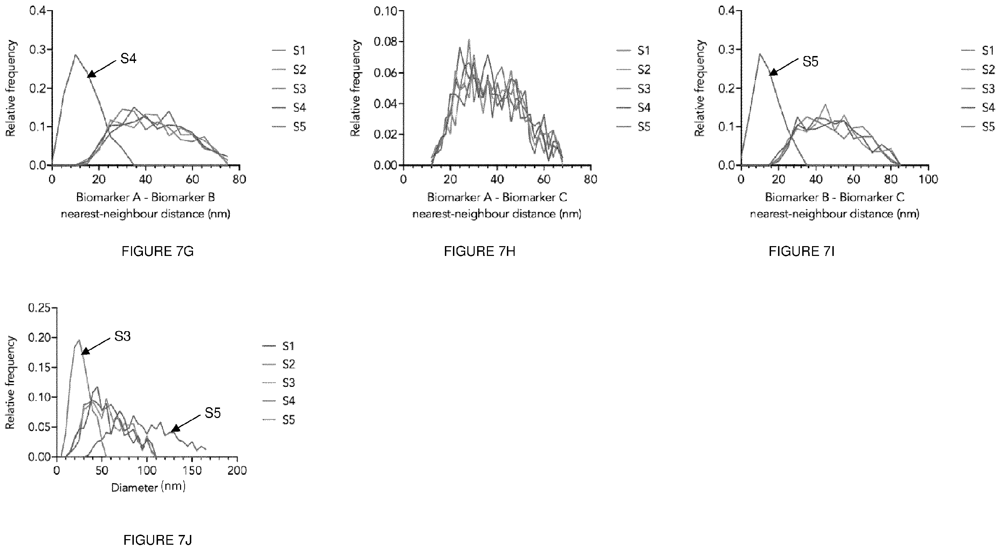

The distributions of the characterising parameters for different populations are shown in Figures 7A-7J. Populations within the "low" or "high" level of a particular characterising parameter are indicated by the arrows in Figures 7A-7E and 7G-7J, and the S5 population falling within the "medium" level for cluster density of C is indicated in Figure 7F.

From these figures, it is evident that the sub-populations would not necessarily be distinguishable by conventional techniques. For example, populations S3 and S4 would be indistinguishable.

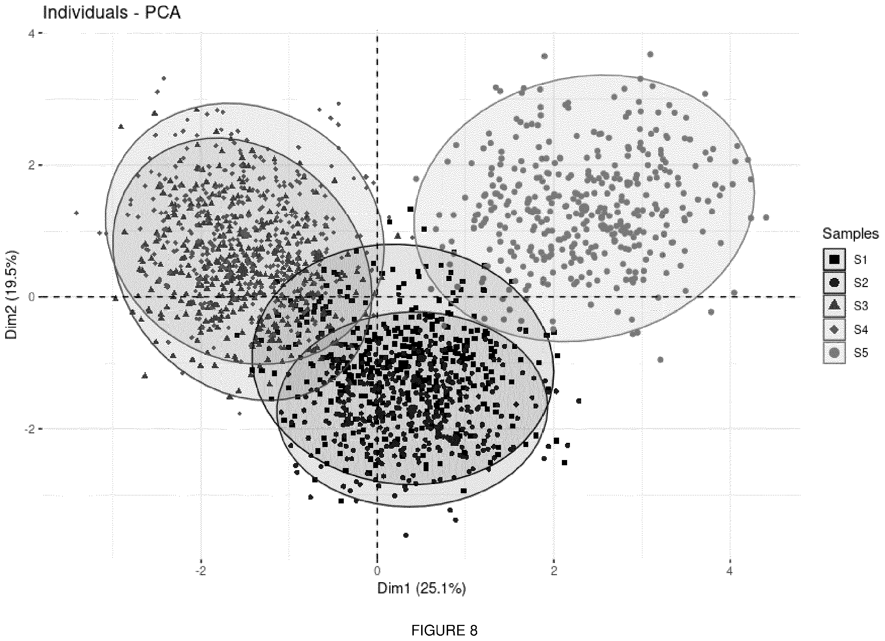

The data sets for S1-S5 were individually subjected to principal component analysis. In each case, the analysis identified the two principal components in which the greatest variance occurs. The results are shown in Figure 8. The data for S5 is relatively well-separated from those for S1-S4, and there is overlap between the data for S1+S2, and S3+S4. From this data, it is evident that the technique would be able to characterise a sample "S6" containing one or a mixture of S1-S5 populations, but with relatively limited ability to distinguish S1 vesicles from S2 vesicles, and likewise to distinguish S3 from S4 vesicles.

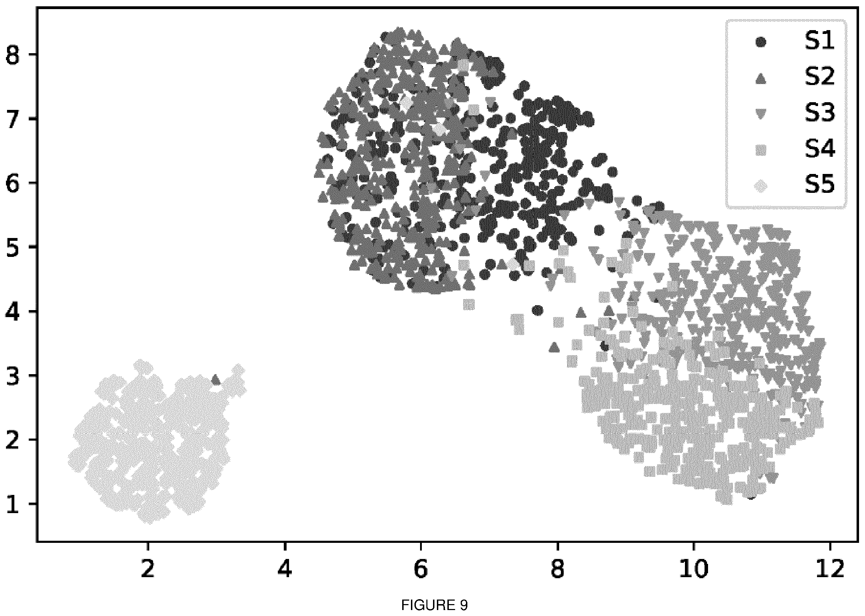

The data set for S1 was then subjected to dimensionality reduction using UMAP, to reduce the feature vectors for individual vesicles to a two-dimensional modified feature vector. The same procedure was repeated for each of S2 to S5. The results of the UMAP analysis are shown in Figure 9. This figure shows the datapoints for S5 clearly separated from the datapoints for S1-S4. Whilst there is some overlap between the S1 and S2 datapoints, as well as the datapoints for S3 and S4, the results show regions with relatively low overlap characteristic of each individual population - more so than in the PCA data shown in Figure 8. These data confirm the ability of the method to take a complex multi-dimensional dataset and synthesise it into a simple to interpret plot. Furthermore, if the analysis were to be repeated with a sample "S6", containing one or a mixture of S1-S5 populations, comparison to the data for populations S1-S5 would allow characterisation of the sample.

### REFERENCES

EV-TRACK Consortium et al. "EV-TRACK: transparent reporting and centralizing knowledge in extracellular vesicle research", Nature Methods (2017), 14, 228-232
Théry, C. et al. "Minimal information for studies of extracellular vesicles 2018 (MISEV2018): a position statement of the International Society for Extracellular Vesicles and update of the MISEV2014 guidelines" J Extracell Vesicles (2018), 7(1), 153750.
Duijvesz, D. et al. "Immuno-based detection of extracellular vesicles in urine as diagnostic marker for prostate cancer", Int. J. Cancer (2015), 15, 137(12), 2569-2878.
Rikkert, L.G. et al. "Detection of extracellular vesicles in plasma and urine of prostate cancer patients by flow cytometry and surface plasmon resonance imaging", PLoS ONE (2020) 15(6), e0233443.
Daaboul, G. G. et al. "Digital Detection of Exosomes by Interferometric Imaging", Scientific Reports (2016), 6, article number 37246.
Saliba et al "Composition and structure of synaptic ectosomes exporting antigen receptor linked to functional CD40 ligand from helper T cells", eLIFE (2019); 8 e47528.
Han, C. et al. "Single-vesicle imaging and co-localization analysis for tetraspaning profiling of individual extracellular vesicles", J Extracell Vesicles (2021) 10: e12047
Lee, K. et al. "Multiplexed Profiling of Single Extracellular Vesicles" ACS Nano (2018), January 12(1), p 494-503
Chang, Y. et al. "Improved resolution in single-molecule localization microscopy using QD-PAINT", Experimental & Molecular Medicine, (2021), 53, 384-392
Joliffe and Cadima, "Principal component analysis: a review and recent developments", Phil. Trans. R. Soc. A (2016) 374: 20150202.
Campello, R. et al. "Density-Based Clustering Based on Hierarchical Density Estimates", Advances in Knowledge Discovery and Data Mining April 2013, pp 160-172.
Schnitzbauer, J. et al "Super-resolution microscopy with DNA-PAINT", Nature Protocols 2017, 12, pp 1198-1228.
Huang et al. "Three-dimensional Super-resolution Imaging by Stochastic Optical Reconstruction Microscopy", Science, 2008, 319(5864), pp 810-813
Li, C. et al "Clinical significance of PD-L1 expression in serum-derived exosomes in NSCLC patients", Journal of Translation Medicine, 2019, 17, Article 355
Gori, A. et al. "Membrane-binding peptides for extracellular vesicles on-chip analysis", J Extracell Vesicles, 2020, 9(1), 1751428
Cnossen, J., et al. "Drift correction in localization microscopy using entropy minimization", Optics Express, 2021, 29(8), pp 27961-27974
Andrews T.S., et al. "Identifying cell populations with scRNASeq", Molecular Aspects of Medicine, 2018, 59, pp 114-122

## Claims

1. A method of characterising vesicles, comprising:
(1) a sample preparation step, comprising providing a test specimen with vesicles attached to a substrate, wherein the vesicles are labelled with one or more fluorescent probes;
(2) an image acquisition step, comprising imaging the vesicles on the test specimen using single molecule localisation microscopy (SMLM) of said one or more fluorescent probes to generate image data including SMLM image data;
(3) an image processing step, comprising:
calculating position data for individual fluorescent probes on the test specimen based on the SMLM image data;
using the position data to identify individual vesicles; and
calculating at least three characterising parameters for individual vesicles from the position data and/or image data, at least one of the characterising parameters being a morphological parameter, and
constructing a feature vector for individual vesicles from the characterising parameters;
(4) a data transformation step, comprising inputting the feature vectors for individual vesicles into a dimensionality reduction algorithm to calculate modified feature vectors of lower dimensionality for individual vesicles; and
(5) a characterisation step, involving characterising each vesicle by comparing the modified feature vector for that vesicle against other modified feature vectors obtained for other vesicles from the test specimen or from reference data.

2. A method according to claim 1, wherein the morphological parameter is derived from said position data for individual fluorescent probes.

3. A method according to claim 2, wherein the morphological parameter is derived from said position data for individual fluorescent probes by identifying ring-shaped accumulations of individual fluorescent probes indicative of the membrane of a vesicle.

4. A method according to claim 2 or 3, wherein the one or more fluorescent probes include a generic fluorescent probe, and said morphological parameter is derived from the position data for said generic fluorescent probe.

5. A method according to any one of claims 1 to 4, wherein the SMLM technique is at least one of (direct) stochastic optical reconstruction microscopy [(d)STORM], photoactivated localisation microscopy (PALM), or point accumulation for imaging in nanoscale topography (PAINT) microscopy, preferably wherein the SMLM technique is fPALM.

6. A method according to any one of the preceding claims, wherein the morphological parameter is one or more of the perimeter of the vesicle and the diameter of the vesicle.

7. A method according to any one of the preceding claims, wherein the characterisation step comprises assigning identified vesicles into two or more sub-populations of vesicles.

8. A method according to any one of claims 1 to 7, wherein the characterisation step comprises characterising each vesicle by comparing the modified feature vector for that vesicle against other modified feature vectors obtained for other vesicles from the test specimen, and comprises assigning identified vesicles into two or more sub-populations of vesicles, preferably wherein assigning identified vesicles into two or more sub-populations comprises carrying out clustering analysis of the modified feature vectors.

9. A method according to any one of the preceding claims, wherein the dimensionality reduction algorithm comprises t-distributed stochastic neighbour embedding (t-SNE), principal component analysis (PCA), or uniform manifold approximation and projection (UMAP), optionally wherein the dimensionality reduction algorithm implements an initial step of PCA followed by t-SNE or UMAP.

10. A method according to any one of the preceding claims, wherein the sample preparation step comprises immobilising the vesicles by providing the surface of the substrate with a binding agent, and contacting the substrate with a vesicle-containing sample such that the vesicles bind to the binding agent.

11. A method according to claim 10, wherein the binding agent comprises or consists of a TIM protein.

12. A method according to any one of the preceding claims, wherein the sample preparation step comprises immobilising the vesicles by:
- treating the substrate with a passivation agent;
- attaching a binding agent to the passivation agent; and
- attaching the vesicles to the substrate through the binding agent.

13. A method according to claim 12, wherein immobilising the vesicles comprises:
i. treating the substrate with a passivation agent to bond the passivation agent to the substrate, wherein at least a fraction of the passivation agent comprises an anchor moiety;
ii. treating the substrate with a mediating compound, the mediating compound having multiple capture moieties suitable for binding to said anchor moiety; and
iii. treating the substrate with a TIM protein, the TIM protein having an anchor moiety which binds to said mediating compound;
wherein preferably the anchor moiety is biotin and the mediating compound is avidin, streptavidin, neutravidin, or a variant thereof.

14. A method according to claim 13, wherein the TIM protein is TIM-4.

15. A method according to any one of the preceding claims, further comprising step (6) a diagnostic step, in which the output from the characterisation step is used to form a clinical picture; optionally wherein the diagnostic step involves identifying a disease state.

## Patentansprüche

1. Verfahren zum Charakterisieren von Vesikeln, umfassend:
(1) einen Probenherstellungsschritt, umfassend das Bereitstellen einer Testprobe mit Vesikeln, die an einem Substrat angebracht sind, wobei die Vesikel mit einer oder mehreren fluoreszierenden Sonden markiert sind;
(2) einen Bildaufnahmeschritt, umfassend das Abbilden der Vesikel auf der Testprobe unter Verwendung von Einzelmolekül-Lokalisierungsmikroskopie (SMLM) der einen oder mehreren fluoreszierenden Sonden, um Bilddaten, einschließlich SMLM-Bilddaten, zu erzeugen;
(3) einen Bildverarbeitungsschritt, umfassend:
Berechnen von Positionsdaten für einzelne fluoreszierende Sonden auf der Testprobe basierend auf den SMLM-Bilddaten;
Verwenden der Positionsdaten, um einzelne Vesikel zu identifizieren; und
Berechnen von zumindest drei kennzeichnenden Paramatern für einzelne Vesikel aus den Positionsdaten und/oder Bilddaten, wobei zumindest einer der kennzeichnenden Parameter ein morphologischer Parameter ist, und
Konstruieren eines Merkmalsvektors für einzelne Vesikel aus den kennzeichnenden Parametern;
(4) einen Datentransformationsschritt, umfassend das Eingeben der Merkmalsvektoren für einzelne Vesikel in einen Dimensionalitätsreduktionsalgorithmus, um modifizierte Merkmalsvektoren niedrigerer Dimensionalität für einzelne Vesikel zu berechnen; und
(5) einen Charakterisierungsschritt, der das Charakterisieren jedes Vesikels durch Vergleichen des modifizierten Merkmalsvektors für jenes Vesikel mit anderen modifizierten Merkmalsvektoren, die für andere Vesikel von der Testprobe oder aus den Referenzdaten erhalten wurden, beinhaltet.

2. Verfahren nach Anspruch 1, wobei der morphologische Parameter von den Positionsdaten für einzelne fluoreszierende Sonden abgeleitet ist.

3. Verfahren nach Anspruch 2, wobei der morphologische Parameter von den Positionsdaten für einzelne fluoreszierende Sonden durch Identifizieren von ringförmigen Akkumulationen von einzelnen fluoreszierenden Sonden, die die Membran eines Vesikels angeben, abgeleitet ist.

4. Verfahren nach Anspruch 2 oder 3, wobei die eine oder die mehreren fluoreszierenden Sonden eine generische fluoreszierende Sonde umfassen und der morphologische Parameter von den Positionsdaten für die generische fluoreszierende Sonde abgeleitet ist.

5. Verfahren nach einem der Ansprüche 1 bis 4, wobei das SMLM-Verfahren zumindest eines aus einer (direkten) stochastischen optischen Rekonstruktionsmikroskopie [(d)STORM], photoaktivierter Lokalisationsmikroskopie (PALM) oder Punktakkumulation zum Abbilden in einer Nanoskalatopographie- (PAINT-) Mikroskopie ist, wobei das SMLM-Verfahren vorzugsweise fPALM ist.

6. Verfahren nach einem der vorangegangenen Ansprüche, wobei der morphologische Parameter einer oder mehrere aus dem Umkreis des Vesikels und dem Durchmesser des Vesikels ist.

7. Verfahren nach einem der vorangegangenen Ansprüche, wobei der Charakterisierungsschritt das Zuweisen identifizierter Vesikel zu zwei oder mehr Subpopulationen von Vesikeln umfasst.

8. Verfahren nach einem der Ansprüche 1 bis 7, wobei der Charakterisierungsschritt das Charakterisieren jedes Vesikels durch Vergleichen des modifizierten Merkmalsvektors für dieses Vesikel mit anderen modifizierten Merkmalsvektoren, die für andere Vesikel von der Testprobe erhalten wurden, umfasst und das Zuweisen identifizierter Vesikel zu zwei oder mehr Subpopulationen von Vesikeln umfasst, wobei das Zuweisen identifizierter Vesikel zu zwei oder mehr Subpopulationen vorzugsweise das Ausführen einer Clusteranalyse der modifizierten Merkmalsvektoren umfasst.

9. Verfahren nach einem der vorangegangenen Ansprüche, wobei der Dimensionalitätsreduktionsalgorithmus eine t-verteilte stochastische Nachbareinbettungs (t-SNE-) Hauptkomponentenanalyse (PCA) oder eine einheitliche Mannigfaltigkeitsannäherung und -projektion (UMAP) umfasst, wobei der Dimensionalitätsreduktionsalgorithmus gegebenenfalls einen anfänglichen Schritt einer PCA, gefolgt von t-SNE oder UMAP, implementiert.

10. Verfahren nach einem der vorangegangenen Ansprüche, wobei der Probenherstellungsschritt das Immobilisieren der Vesikel durch Bereitstellen der Oberfläche des Substrats mit einem Bindemittel und das Kontaktieren des Substrats mit einer Vesikel-enthaltenden Probe umfasst, sodass die Vesikel an das Bindemittel binden.

11. Verfahren nach Anspruch 10, wobei das Bindemittel ein TIM-Protein umfasst oder daraus besteht.

12. Verfahren nach einem der vorangegangenen Ansprüche, wobei der Probenherstellungsschritt das Immobilisieren der Vesikel durch
- Versetzen des Substrats mit einem Passivierungsmittel;
- Binden eines Bindemittels an das Passivierungsmittel; und
- Binden der Vesikel durch das Bindemittel an das Substrat umfasst.

13. Verfahren nach Anspruch 12, wobei das Immobilisieren der Vesikel Folgendes umfasst:
i. Versetzen des Substrats mit einem Passivierungsmittel, um das Passivierungsmittel mit dem Substrat zu verbinden, wobei zumindest ein Teil des Passivierungsmittels eine Ankergruppierung umfasst;
ii. Versetzen des Substrats mit einer vermittelnden Verbindung, wobei die vermittelnde Verbindung mehrere Einfanggruppierungen aufweist, die zur Bindung an die Ankergruppierung geeignet sind; und
iii. Versetzen des Substrats mit einem TIM-Protein, wobei das TIM-Protein eine Ankergruppierung aufweist, die an die vermittelnde Verbindung bindet;
wobei vorzugsweise die Ankergruppierung Biotin ist und die vermittelnde Verbindung Avidin, Streptavidin, Neutravidin oder eine Variante davon ist.

14. Verfahren nach Anspruch 13, wobei das TIM-Protein TIM-4 ist.

15. Verfahren nach einem der vorangegangenen Ansprüche, das weiters Schritt (6) als Diagnoseschritt umfasst, in dem die Ausgabe von dem Charakterisierungsschritt verwendet wird, um ein klinisches Bild zu bilden; wobei der Diagnoseschritt gegebenenfalls das Identifizieren eines Erkrankungszustands beinhaltet.

## Revendications

1. Procédé de caractérisation de vésicules, comprenant :
(1) une étape de préparation d'échantillon, consistant à fournir un échantillon de test avec des vésicules fixées à un substrat, dans lequel les vésicules sont marquées avec une ou plusieurs sondes fluorescentes ;
(2) une étape d'acquisition d'image, consistant à imager des vésicules sur l'échantillon de test en utilisant une microscopie de localisation à molécule unique (SMLM) desdites une ou plusieurs sondes fluorescentes pour générer des données d'image incluant des données d'image SMLM ;
(3) une étape de traitement d'image, consistant à :
calculer des données de position pour des sondes fluorescentes individuelles sur l'échantillon de test sur la base des données d'image de SMLM ;
utiliser les données de position pour identifier des vésicules individuelles ; et
calculer au moins trois paramètres de caractérisation pour des vésicules individuelles à partir des données de position et/ou des données d'image, au moins un des paramètres de caractérisation étant un paramètre morphologique, et
construire un vecteur de caractéristiques pour les vésicules individuelles à partir des paramètres de caractérisation ;
(4) une étape de transformation de données, consistant à entrer les vecteurs de caractéristiques pour des vésicules individuelles dans un algorithme de réduction de dimensionnalité afin de calculer les vecteurs de caractéristiques modifiés de dimensionnalité inférieure pour des vésicules individuelles ; et
(5) une étape de caractérisation, consistant à caractériser chaque vésicule en comparant le vecteur de caractéristiques modifié pour cette vésicule à d'autres vecteurs de caractéristiques modifiés obtenus pour d'autres vésicules à partir de l'échantillon de test ou de données de référence.

2. Procédé selon la revendication 1, dans lequel le paramètre morphologique est dérivé desdites données de position pour des sondes fluorescentes individuelles.

3. Procédé selon la revendication 2, dans lequel le paramètre morphologique est dérivé desdites données de position pour des sondes fluorescentes individuelles en identifiant des accumulations en forme d'anneau de sondes fluorescentes individuelles indicatives de la membrane d'une vésicule.

4. Procédé selon la revendication 2 ou 3, dans lequel les une ou plusieurs sondes fluorescentes incluent une sonde fluorescente générique, et ledit paramètre morphologique est dérivé des données de position pour ladite sonde fluorescente générique.

5. Procédé selon l'une quelconque des revendications 1 à 4, dans lequel la technique de SMLM est au moins l'une de la microscopie de reconstruction optique stochastique (directe) [(d)STORM], de la microscopie de localisation photoactivée (PALM) ou de l'accumulation de points pour l'imagerie en microscopie en topographie à l'échelle nanométrique (PAINT), de préférence dans lequel la technique de SMLM est fPALM.

6. Procédé selon l'une quelconque des revendications précédentes, dans lequel le paramètre morphologique est un ou plusieurs parmi le périmètre de la vésicule et le diamètre de la vésicule.

7. Procédé selon l'une quelconque des revendications précédentes, dans lequel l'étape de caractérisation comprend l'attribution de vésicules identifiées dans deux sous-populations de vésicules ou plus.

8. Procédé selon l'une quelconque des revendications 1 à 7, dans lequel l'étape de caractérisation comprend la caractérisation de chaque vésicule en comparant le vecteur de caractéristiques modifié pour cette vésicule à d'autres vecteurs de caractéristiques modifiés obtenus pour d'autres vésicules à partir de l'échantillon de test, et comprend l'attribution de vésicules identifiées dans deux sous-populations de vésicules ou plus, de préférence dans lequel l'attribution de vésicules identifiées dans deux sous-populations ou plus comprend la réalisation d'une analyse de regroupement des vecteurs de caractéristiques modifiés.

9. Procédé selon l'une quelconque des revendications précédentes, dans lequel l'algorithme de réduction de dimensionnalité comprend un encastrement de voisin stochastique distribué en t (t-SNE), une analyse en composantes principales (PCA) ou une approximation et une projection uniforme ( de variétés (UMAP), facultativement dans lequel l'algorithme de réduction de dimensionnalité met en œuvre une étape initiale de PCA suivie de t-SNE ou de UMAP.

10. Procédé selon l'une quelconque des revendications précédentes, dans lequel l'étape de préparation d'échantillon comprend l'immobilisation des vésicules en dotant la surface du substrat d'un agent de liaison, et la mise en contact du substrat avec un échantillon contenant des vésicules de telle sorte que les vésicules se lient à l'agent de liaison.

11. Procédé selon la revendication 10, dans lequel l'agent de liaison comprend ou est constitué d'une protéine TIM.

12. Procédé selon l'une quelconque des revendications précédentes, dans lequel l'étape de préparation d'échantillon comprend l'immobilisation des vésicules par l'intermédiaire des étapes consistant à :
- traiter le substrat avec un agent de passivation ;
- fixer un agent de liaison à l'agent de passivation ; et
- fixer les vésicules au substrat par l'intermédiaire du liant.

13. Procédé selon la revendication 12, dans lequel l'immobilisation des vésicules comprend les étapes consistant à :
i. traiter le substrat avec un agent de passivation pour lier l'agent de passivation au substrat, dans lequel au moins une fraction de l'agent de passivation comprend une fraction d'ancrage ;
ii. traiter le substrat avec un composé médiateur, le composé médiateur présentant de multiples fractions de capture appropriées pour se lier à ladite fraction d'ancrage ; et
iii. traiter le substrat avec une protéine TIM, la protéine TIM présentant une fraction d'ancrage qui se lie audit composé médiateur ;
dans lequel, de préférence, la fraction d'ancrage est la biotine et le composé médiateur est l'avidine, la streptavidine, la neutravidine, ou une variante de celles-ci.

14. Procédé selon la revendication 13, dans lequel la protéine TIM est TIM-4.

15. Procédé selon l'une quelconque des revendications précédentes, comprenant en outre une étape (6) de diagnostic, dans laquelle la sortie de l'étape de caractérisation est utilisée pour former un tableau clinique ; facultativement dans lequel l'étape de diagnostic implique l'identification d'un état pathologique.
